# EUROPEAN PATENT APPLICATION

(11) **EP 1 645 563 A1**
(43) Date of publication of application: **12.04.2006**
(21) Application number: 04747133.9
(22) Date of filing: 07.07.2004
(51) Int. Cl.: C07K 1/113

(54) **PROTEIN-REFOLDING MATERIAL**

(30) Priority: 09.07.2003 JP 2003272398; 05.01.2004 JP 2004000535; 05.01.2004 JP 2004000711; 31.03.2004 JP 2004101710
(71) Applicant: National Institute of Advanced Industrial Science and Technology, Tokyo 100-8921 (JP)
(72) Inventor: MIZUKAMI, Fujio, Nat. Inst. of Adv. Ind. Sci.&Tec., Sendai-shi, Miyagi 983-8551 (JP); KIYOZUMI, Y., Nat. Inst. of Adv. Ind. Sci. & Tec., Sendai-shi, Miyagi 983-8551 (JP); IKEDA, Takuji, Nat. Inst. of Adv. Ind. Sci. & Tec., Sendai-shi, Miyagi 983-8551 (JP); KAWAI, Akiko, Nat. Inst. of Adv. Ind. Sci. & Tec., Sendai-shi, Miyagi 983-8551 (JP); NAGASE, Takako, Nat. Inst. of Adv. Ind. Sci.&Tec., Sendai-shi, Miyagi 983-8551 (JP); SAKAGUCHI, Kengo, Tsukuba-shi, Ibaraki 3002667 (JP); CHIKU, Hiroyuki, Yokohama-shi, Kanagawa 2400035 (JP)
(74) Representative: Ebner von Eschenbach, Jennifer
(86) International application number: PCT/JP2004/009664
(87) International publication number: WO 2005/005459

(57) **Abstract**

The present invention provides a method for refolding protein produced, for example, by Escherichia coli, that is inactive due to an as yet unformed higher order structure, or protein deactivated due to a change in conformation for some reason. The invention comprises a method, refolding kit, refolding agent, and molding that activate a native function or activity inherent to a protein through treatment with zeolite beta of protein produced, for example, by Escherichia coli, that is inactive due to an as yet unformed higher order structure, or protein deactivated due to a change in conformation for some reason. The invention also comprises a method for producing an active protein that utilizes the same. As compared with conventional methods, the present invention can provide a novel method for activating protein function that is highly versatile and generalizable, that employs a simple and easy protocol, and that is inexpensive and enables repeated use of the function activator.

## Description

### TECHNICAL FIELD

This invention relates to a method for activating the function of inactive protein, and more particularly relates to a method for activating the function of inactive protein by inducing the refolding of a protein that is inactive because its higher order structure has yet to be formed or protein that is deactivated due to a change in conformation for some reason, thereby enabling activation or regeneration of a native function inherent to the protein. This invention also relates to a method for producing an active protein utilizing this method for activating the function of inactive protein. The invention further relates to reagents, substances, and materials used in protocols and steps for activating the function of inactive protein, and more particularly relates to reagents, substances, and materials, a so-called reagent kit, used in protocols and steps for inducing the refolding of a protein that is inactive because its higher order structure has yet to be formed or a protein that is deactivated due to a change in conformation for some reason, thereby enabling activation or regeneration of a native function inherent to the protein. The invention also relates to the activation of inactive protein using such a kit. The invention additionally relates to a function activator (refolding agent) and a function-activating molding for inactive protein and more particularly relates to certain types of devices, such as chips, that have the capacity to induce the refolding of a protein that is inactive because its higher order structure has yet to be formed or a protein that is deactivated due to a change in conformation for some reason, thereby activating or regenerating a native function inherent to the protein. The invention also relates to the activation of inactive protein, that is, the production or generation of active protein, using such a device.

### BACKGROUND ART

The actual processes and functions within living organisms are not carried out by genes, but rather by the proteins produced from genes. The elucidation and analysis of protein structure and function is thus directly related to, for example, drugs and disease treatment, and is therefore of vital importance. As a consequence, efforts are actively underway with regard to the synthesis and production of various proteins by a variety of methods, the investigation of the structures of these proteins, and the elucidation of their action mechanism and role in living organisms. As is well known at this time, protein function is determined not only by the sequence and chain length of the amino acids that make up a protein, but also by the ordered conformation (higher order structure) assumed by the protein.

Protein synthesis is generally carried out using, for example, an Escherichia coli, insect cell, or mammalian cell expression system. Synthesis using insect or mammalian cells very often yields soluble protein with a controlled higher order structure that assumes an orderly conformation. The separation and purification processes in these methods, however, are very complex. Not only is recovery of the target protein time consuming and expensive, but very little protein is obtained. In contrast, protein synthesis by Escherichia coli involves simple procedures, does not require a great deal of time to obtain the target protein, and also is not very expensive. As a consequence, methods that use Escherichia coli that incorporates the genetic code responsible for synthesis of a target protein have at the present time become the mainstream for protein synthesis and the corresponding production processes are also being established.

However, when protein from a higher organism such as humans is synthesized using an Escherichia coli expression system, the intended protein is in fact obtained in terms of the number of amino acids and their bonding sequence, i.e., in terms of the amino acid chain, but the obtained protein has a disordered conformation and an uncontrolled higher order structure, that is, an insoluble protein is obtained, known as an inclusion body, in which the amino acid chain is entangled. This inclusion body of insoluble protein naturally lacks the desired functions and properties and lacks activity. As a result, an Escherichia coli-based production process requires refolding of the inclusion body, that is, a process in which the inclusion body is unraveled and converted into soluble protein with a modulated higher order structure and an orderly conformation.

This type of refolding is applied not only to protein produced by Escherichia coli, but is also applicable to the regeneration of protein that is deactivated by certain mechanisms, for example, the thermal history, and is therefore an extremely important technology. This refolding has thus been under very active investigation, and, while different methods have been proposed, almost all of these methods have a low refolding rate and frequently can do nothing more than sporadically give desirable results for certain limited proteins (specific low molecular weight proteins in particular). At present there is no method for carrying out this refolding that is economical and efficient, that provides a high refolding rate, and that is a versatile and general method applicable to variety of proteins.

Dialysis and dilution are the refolding techniques that have long been in the most frequent use. In the former technique, the protein is dissolved in an aqueous solution that contains detergent and/or denaturant, and this is dialyzed with buffer lacking detergent and denaturant in order to reduce the detergent and/or denaturant concentrations and refold the protein (typical example: the FoldIt Kit from Hampton Research Corporation). In the latter technique, the protein is dissolved in an aqueous solution containing detergent and/or denaturant and this is simply diluted in order to reduce the detergent and/or denaturant concentrations and induce refolding (typical example: the FoldIt Kit from Hampton Research Corporation). While these are the techniques in general use, there are also other methods for inducing refolding using a diluent, for example, a refolding method in which a glutathione S-transferase fusion protein is dissolved in a solution of sodium N-lauroyl sarcosinate detergent and this is diluted with 1 to 2% Triton X-100 (refer to Anal. Biochem. Vol. 210 (1993) 179-187).

A consumable kit is commercially available from Hampton Research Corporation for both dialysis and dilution. Nothing more has been seen for these protocols than the generation of refolding for a very limited number of proteins, such as ligand binding domains for glutamate and kainite receptors, lysozyme, and carbonic anhydrase B (refer to Protein Sci. Vol. 8 (1999): 1475-1483), and it is no exaggeration to say that they remain in the realm of trial and error methods. Thus, even when a method occasionally goes smoothly, it is quite often the case that it almost never goes well when applied to another protein.

The use of an adsorption separation column for refolding has also been attempted. Refolding is produced during gel filtration when thioredoxin protein denatured by guanidine hydrochloride or urea is subjected to gel filtration (refer to Biochemistry, Vol. 26 (1987) 3135-3141). However, refolding by this method is not always satisfactory, and satisfactory results are usually not obtained with other proteins. The eluted protein is refolded when protein solubilized with 8 M urea is adsorbed on a column on which the molecular chaperone GroEL (a molecular chaperone is a type of protein that promotes the refolding of structure-disrupted protein) has been immobilized and is eluted with a solution that contains 2 M potassium chloride and 2 M urea (refer to Proc. Natl. Acad. Sci. USA, Vol. 94 (1997) 3576-3578). However, this is confined to a demonstration for a very limited number of proteins, such as cyclophilin A. In particular, the use of molecular chaperones involves a certain type of template, and this approach is in fact completely useless for material not conforming to the shape of this template.

Protein refolding on the resin has also been reported to occur when guanidine hydrochloride-denatured scorpion toxin Cn5 protein is mixed with resin on which three proteins thought to be related to refolding promotion (GroEL, disulfide oxidereductase from Escherichia coli (DsbA), and human proline cis-trans isomerase (PPI)) are simultaneously immobilized (refer to Nat. Biotechnol. Vol. 17 (1999) 187-191). However, in addition to the drawback that this approach can be applied only to specific proteins, such as scorpion toxin Cn5, the preparation of the three protein-functionalized resin is complex and expensive.

Metal chelates have also been used in place of refolding protein as the material immobilized on a column. His6-tagged fusion protein undergoes refolding when, after dissolution and denaturation with an aqueous solution containing guanidine hydrochloride and urea and adsorption onto a resin on which a nickel chelate has been immobilized, washing is carried out with a buffer solution lacking denaturant (Life Science News (Japan Ed.) Vol. 3 (2001) 6-7). Again, the application of this method is limited to this protein and preparation of the resin is complex and expensive.

Protein refolding has also been reported with the use of beta-cyclodextrin and cycloamylose as artificial chaperones. When detergent-denatured protein is mixed into a solution of such a chaperone, the detergent is incorporated and sequestered by the artificial chaperone and the protein undergoes refolding during this process (J. Am. Chem. Soc. Vol. 117 (1995) 2373-2374; J. Biol. Chem. Vol. 271 (1996) 3478-3487; and FEBS Lett. Vol. 486 (2000) 131-135). The success of this method, however, is confined to, for example, carbonic anhydrase B. Moreover, it is not a method that can be carried out repetitively and it is therefore expensive.

The inventors, on the other hand, have continued to carry out research up to the present time on the adsorption of biopolymers to zeolites (Chem. Eur. J., Vol. 7 (2001) 1555-1560), such as ZSM zeolite and zeolite beta (for example, refer to Zeolites, Vol. 11 (1991) 842-845; Adv. Mater., Vol. 8 (1996) 517-520; Japanese Laid-Open Patent Application No. H06-127937; and Japanese Laid-Open Patent Application No. H08-319112).

### DISCLOSURE OF THE INVENTION

While a variety of refolding methods have already been reported as described above, the problems cited above are also associated with these methods, and a pressing issue in this area of technology has therefore been the development of a highly efficient, low-cost refolding method that enables repeated use, that is very versatile and generalizable, and that can be applied, regardless of chain length, to a variety of proteins that have either been denatured and deactivated or whose higher order structure has yet to be formed. In addition, while a variety of refolding methods as well as substances and materials that have a refolding activity have already been reported and refolding kits made therefrom have already been commercialized, the problems cited above are nevertheless still associated with these methods, substances, materials, and kits, and a pressing issue in this area of technology has therefore been the development of a highly efficient, low-cost refolding substance, material, and method that enable repeated use, that are very versatile and generalizable, and that can be applied, regardless of chain length, to a variety of proteins that have either been denatured and deactivated or whose higher order structure has yet to be formed, in other words, the development of an economical, high-performance refolding technology and refolding kit based thereon. Moreover, while a variety of refolding methods as well as substances and materials that have a refolding activity have been reported to date, these still suffer from the various problems described hereinabove, such as a lack of generalizability, cumbersome procedures, and high cost. Due to this, a pressing issue in this area of technology has therefore been, first of all, the development of a highly efficient, low-cost refolding substance, material, and method that enable repeated use, that are very versatile and generalizable, and that can be applied, regardless of chain length, to a variety of proteins that have either been denatured and deactivated or whose higher order structure has yet to be formed. A second major issue has been that the prior refolding protocols and processes have required the use of a centrifugal separator and chromatography, and the repeated use thereof makes these protocols and processes complex and cumbersome, very time consuming, and expensive.

Against these circumstances and in view of the prior art as described above, the inventors carried out focused research and development for the purpose of developing a novel refolding technology capable of solving the problems described hereinabove and also carried out detailed investigations into the nature of the adsorption of biopolymers, e.g., DNA, RNA, protein, to metal oxides, such as zeolites (Chem. Eur. J. Vol. 7 (2001) 1555-1560), as well as focused research on methods for the separation and purification of protein. As a result of these campaigns, the inventors discovered that when protein produced, for example, by an Escherichia coli expression system, with an as yet unformed higher order structure, or protein deactivated for some reason, such as its thermal history, is treated with zeolite beta, such protein will exhibit its native function and activity. The inventors also discovered that this method can be used as a highly versatile, highly generalizable method according to the present invention that is applicable to the refolding of a variety of conformation-disordered proteins, including large proteins with molecular weights in excess of 100,000. This invention was achieved based on these discoveries. The inventors also discovered that zeolite with the BEA structure, that is, zeolite beta, has a refolding activity for denatured protein, and concomitant with the development of a refolding agent comprising zeolite beta, the inventors also developed a protein refolding kit in which this refolding agent is an essential constituent component. It was additionally found that this refolding agent can also be applied to the refolding of a variety of conformation-disordered proteins, including large proteins with molecular weights in excess of 100,000, such as protein produced, for example, by an Escherichia coli expression system, with an as yet unformed higher order structure, or protein deactivated for some reason, such as its thermal history, and this invention, that is, a highly versatile and highly generalizable protein refolding technology and refolding kit, was achieved thereby. An object of a first aspect of this invention is to provide a method for activating protein function. An object of a second aspect of this invention is to provide a novel refolding kit. An object of a third aspect of this invention is to provide a novel protein refolding material. An object of a fourth aspect of this invention is to provide a refolding molding comprising a molding that contains zeolite with the BEA structure and to provide a refolding molding that, by inducing the refolding of inactive protein, has the capacity to activate or regenerate a native function inherent to the protein.

A first aspect of the present invention is described in additional detail below.

The first aspect of the present invention comprises the following technical means.
(1) A method of activating the function of a protein that is inactive due to a disordered higher order structure, comprising bringing the protein into a state that can express a native function inherent to the protein, by bringing the protein into contact with zeolite beta.
(2) The method according to (1), wherein the protein is brought into contact with the zeolite beta in the presence of a protein denaturant, a surfactant, and/or a refolding buffer.
(3) The method according to (1), wherein the protein that is inactive due to a disordered higher order structure is a protein that is produced by an Escherichia coli expression system.
(4) The method according to (1), wherein the protein that is inactive due to a disordered higher order structure is a protein that is deactivated due to its thermal history.
(5) The method according to (1), wherein the protein is adsorbed to the zeolite beta by mixing with a solution that contains the zeolite beta or by introduction onto a column packed with the zeolite beta and is then desorbed from the zeolite beta.
(6) A method for reforming the core structure of a protein, comprising refolding the conformation of a protein that is inactive due to a disordered higher order structure by bringing the protein into contact with zeolite beta.
(7) A method for producing an active protein, comprising refolding the conformation of a protein that is inactive due to a disordered higher order structure by bringing the protein into contact with zeolite beta, thereby producing a protein that has a controlled higher order structure and an activated native function inherent to the protein.
(8) The method according to (7) for producing a protein, comprising refolding the conformation of an inactive protein produced by Escherichia coli that incorporates the genetic code responsible for the synthesis of a target protein, by bringing the inactive protein into contact with zeolite beta.

Protein for submission to function activation according to the present invention generally comprises conformation-disordered protein produced by, for example, an Escherichia coli expression system and known as an inclusion body, as well as protein deactivated for some reason, such as the thermal history. In accordance with the present invention, a native function inherent to this protein is activated by refolding the conformation of the protein by treating the protein with zeolite beta. The activation protocol is typically carried out by first dispersing and dissolving the protein in a solution containing, for example, denaturant and/or detergent (surfactant); thereafter adsorbing the protein to zeolite beta by mixing with a zeolite beta-containing solution or by introduction onto a column packed with zeolite beta; and then desorbing the protein from the zeolite beta. The zeolite beta used as the function activator in the present invention can be exemplified by uncalcined zeolite beta and by calcined zeolite beta obtained by the calcination of synthetic zeolite beta for 3 to 10 hours at 300 to 500°C. However, the invention is not limited to these and zeolite beta equivalent to the preceding can be similarly used.

Given that in general the protein is frequently produced in, for example, an Escherichia coli expression system, and is typically frequently used in aqueous solution, and that the deactivated protein frequently resides in aqueous solution, water, for example, is very suitably used as the solvent for dispersing the protein prior to adsorption to the zeolite beta. However, the solvent is not necessarily limited to this, and solvents can be used, either as such or mixed with water, that do not react with the protein or cause the conformation of the protein to change to an unintended shape and thus that are basically free of problems. Typical examples of solvents of this type are monovalent and polyvalent alcohols, but there is no restriction to these.

The subject protein adsorption/desorption is generally carried out in the presence of denaturant and/or detergent, pH regulator, refolding factor, and so forth in order to facilitate unraveling of the entangled protein chain, e.g., an inclusion body, and facilitate its refolding, and/or in the presence of some type of reducing agent in order to cleave S-S bonds unintentionally formed in the protein chain. Typical examples of these denaturants and/or detergents, pH regulators, and refolding factors are guanidine hydrochloride, trisaminomethane hydrochloride, polyethylene glycol, cyclodextrin, 4-(2-hydroxyethyl)-1-piperazineethanesulfonic acid (HEPES), polyphosphoric acid, sucrose, glucose, glycerol, inositol, Dextran T-500, and Ficol1400, but these substances are not confined to the preceding and any substance that has an equivalent action can be used.

2-mercaptoethanol, because it is inexpensive and easy to acquire, is typically used as the reducing agent that supports return to the native structure by cleavage of unintentionally formed S-S bonds; however, this reducing agent is not limited thereto and any substance that has an equivalent activity can be used. Of course, when unraveling of the protein chain proceeds easily or when there is no unintended S-S bond production, use of the denaturant and/or detergent and/or inhibitor is not necessarily required and their presence therefore is not always required; rather, their use should be selected as appropriate in correspondence to the circumstances. When these substances are used, their quantities are determined as appropriate to the circumstances.

Displacement adsorption is generally used for desorption of the protein; however, there are no particular limitations here as basically any procedure can be used that does not impair activation of the function after desorption of the protein. Thus, changes in the pH or temperature can also be used, and these can also be used in combination with displacement adsorption. A detergent such as sodium dodecyl sulfate (SDS) or a salt such as an alkali halide is generally used as the substance that induces desorption of the protein by displacement adsorption, but there is no limitation to these. Insofar as there is no impairment of activation of the function after desorption of the protein, a variety of substances can generally be used, such as the substances used for elution in column chromatography.

Various supplementary procedures can also be carried out in combination with the aforementioned protocol in order to induce adsorption of the protein to the silicate or desorption therefrom. A typical example of such a procedure involves, for example, exposure to ultrasound or microwaves and/or application of a magnetic or electrical field. The procedures and protocol according to the present invention as described above cause the refolding of protein produced using, for example, an Escherichia coli expression system, that has an as yet unformed higher order structure, and cause the refolding of protein deactivated for some reason, and thereby rapidly activate a native function of such proteins. The zeolite beta function activator according to the present invention is very stable both thermally and chemically and is inexpensive and in addition can be used repeatedly. This invention is extremely useful for the production of biochemical and pharmaceutical products and has immeasurable economic effects.

A second aspect of the present invention is described in additional detail below.

The second aspect of the present invention comprises the following technical means.
(1) A protein refolding kit that is a reagent kit used in a protein function activation (refolding) protocol or step that modulates the higher order structure of a protein that is inactive due to a disordered higher order structure, thereby activating the protein, wherein the protein refolding kit contains a refolding agent comprising zeolite with the BEA structure (zeolite beta) as a constituent.
(2) The refolding kit according to (1), wherein the kit has protein denaturant, pH regulator, and refolding agent comprising the aforementioned zeolite beta as basic constituent components and additionally comprises a combination that contains at least one selection from agents that inhibit the formation of protein S-S bridges, surfactants, and refolding factors.
(3) The refolding kit according to (1) or (2), wherein the framework structure of the zeolite beta contains silicon, oxygen, and at least one element other than silicon and oxygen.
(4) The refolding kit according to any of (1) to (3), wherein the framework structure of the zeolite beta comprises only silicon and oxygen or only silicon and aluminum and oxygen.
(5) The refolding kit according to any of (1) to (4), wherein the zeolite beta contains an ammonium species.
(6) The refolding kit according to (5), wherein the ammonium species is ammonium ion, an organic amine, and/or an acid amide.
(7) The refolding kit according to (6), wherein the organic amine is a tetraalkylammonium.
(8) The refolding kit according to any of (2) to (7), wherein the protein denaturant in the kit is guanidine hydrochloride.
(9) The refolding kit according to any of (2) to (8), wherein the pH regulator in the kit is trisaminomethane trihydrochloride (TrisHCl) and/or 4-(2-hydroxyethyl)-1-piperazineethanesulfonic acid (HEPES).
(10) The refolding kit according to any of (2) to (9), wherein the agent in the kit that inhibits the formation of protein S-S bridges is 2-mercaptoethanol, dithiothreitol, cystine, or thiophenol.
(11) The refolding kit according to any of (2) to (10), wherein the surfactant and refolding factor in the kit are at least one selection from polyethylene glycol, Ficol170, Ficol1400, polyphosphoric acid, sodium dodecyl sulfate (SDS), sucrose, glucose, glycerol, inositol, cyclodextrin, amylose, Dextran T-500, Tween 20, Tween 40, Tween 60, NP-40, SB3-14, SB12, CTAB, and Triton X-100.
(12) The refolding kit according to any of (1) to (7), wherein the kit comprises the aforementioned refolding agent, guanidine hydrochloride, TrisHCl, 2-mercaptoethanol, and a solution (the refolding buffer) comprising HEPES, alkali halide, 2-mercaptoethanol, refolding factor, and surfactant, or the kit comprises the refolding buffer, the refolding agent, guanidine hydrochloride, TrisHCl, 2-mercapethanol, and alkali halide.

Protein that may be processed by the reagent set, that is, the refolding kit, according to the present invention for activation of a function of an inactive protein is any inactive protein with an irregular higher order structure, but generally will be conformation-disordered protein produced by, for example, an Escherichia coli expression system and known as an inclusion body, or protein deactivated for some reason, such as the thermal history. The kit according to the present invention effects activation or generation of a native function of a protein by refolding the conformation of the protein by a process in which the protein is adsorbed to and desorbed from a refolding agent comprising zeolite beta that is present in the kit. However, the subject capability of the refolding agent is not necessarily limited to the preceding and is generally manifested by the following protocol. In other words, activation of a function of an inactive protein is carried out by the following protocol. That is, this protocol is carried out by a sequence in which the protein is first dissolved and dispersed in a solution containing denaturant and/or detergent (surfactant) and so forth; this is mixed with a solution containing the refolding agent, or is introduced onto a column packed with the refolding agent, in order to adsorb the protein to the refolding agent; and the protein is then desorbed from the refolding agent.

In addition to the refolding agent comprising zeolite beta, the kit according to the present invention suitably contains, for example, denaturant and/or pH regulator and, in addition to the preceding, comprises refolding factor and/or detergent and S-S bridge formation inhibitor in order to prevent reformation of the inclusion body after refolding and promote desorption of the protein from the refolding agent.

A typical example of this type of denaturant in the kit according to the present invention is guanidine hydrochloride, while typical examples of the pH regulator in the kit according to the present invention are trisaminomethane hydrochloride (TrisHCl) and 4-(2-hydroxyethyl)-1-piperazineethanesulfonic acid (HEPES). However, the kit according to the present invention is not limited to these, and any substance with the equivalent activity can be used.

Typical examples of the refolding factor and detergent in the kit according to the present invention are polyethylene glycol (PEG20K, PEG800, PEG200, PEG3350), Ficol170, Ficol1400, polyphosphoric acid, sodium dodecyl sulfate (SDS), sucrose, glucose, glycerol, inositol, cyclodextrin, amylose, Dextran T-500, Tween 20, Tween 40, Tween 60, NP-40, SB3-14, SB12, CTAB, and Triton X-100. However, the kit according to the present invention is not limited to these, and any substance with the equivalent activity can be used.

Of course, when unraveling of the protein chain proceeds easily or when there is no unintended S-S bridge production, use of the denaturant and/or detergent and/or inhibitor is not necessarily required and their presence therefore is not always required; rather, their use in the kit should be selected as appropriate in correspondence to the circumstances of use. In addition, the amounts of the particular components making up the kit are determined as appropriate for the circumstances. The kit according to the present invention preferably generally contains refolding agent comprising zeolite beta, guanidine hydrochloride (denaturant), and TrisHCl and HEPES (pH regulators) and also one or more selections from 2-mercaptoethanol and the aforementioned refolding factors and detergents.

In general, displacement adsorption by a reagent (refolding factor and/or detergent) present in the subject kit is ordinarily used for protein desorption during refolding procedures and operations using the kit. However, basically any procedure that does not impair activation of the function after protein desorption is usable here and there are otherwise no particular restrictions thereon. Thus, changes in the pH or temperature can be used, and these can also be used in combination with displacement adsorption.

The salts, e.g., alkali halides, heretofore used for elution in column chromatography are frequently used as a substance that induces desorption of the protein during displacement adsorption, and remarkable results are frequently also obtained by the co-use thereof. Moreover, with regard to actual use of the reagents making up the kit according to the present invention, some of the kit reagents can also be combined in advance to make a single reagent, insofar as there are no particular problems or obstacles, such as the occurrence of a reaction between or among the kit reagents. For example, a single solution comprising HEPES, alkali halide (for example, sodium chloride), 2-mercaptoethanol, refolding factor (for example, beta-cyclodextrin or Ficol170 and/or a selection from the polyethylene glycols PEG20K, PEG800, PEG200, and PEG3350), and detergent (for example, Tween 20, Tween 40, Tween 60, NP-40, or Triton X-100) can be made up as a refolding buffer and this can also be provided as one of the reagents making up the kit according to the present invention. This is quite often very convenient for the refolding protocol and steps.

Various supplementary procedures can also be carried out in combination with the aforementioned protocol in order to induce adsorption of the protein to the refolding agent in the kit or desorption therefrom. A typical example of such a procedure involves, for example, exposure to ultrasound or microwaves and/or application of a magnetic or electrical field. The aforementioned procedures and protocol using the kit according to the present invention cause the protein refolding capacity of the subject refolding agent to be strongly expressed, resulting in the refolding of protein produced using, for example, an Escherichia coli expression system, that has an as yet unformed higher order structure, or the refolding of protein deactivated for some reason, and thereby rapidly activating functionality that should be native to such proteins.

Typical examples of the zeolite with the BEA structure, that is, zeolite beta, that constitutes the refolding agent which expresses an activation function for inactive protein and a refolding capacity for denatured protein are the usual commercially available zeolite betas (for example, HSZ-930NHA from Tosoh Corporation); zeolite beta as synthesized or produced inhouse in accordance with directions found in the literature (refer to Zeolites, Vol. 11 (1991) 202); zeolite beta obtained by calcination of the preceding examples; zeolite beta in which an ammonium species (e.g., ammonium, various aliphatic and/or aromatic ammonium species) resides in the zeolite cavities; framework-substituted zeolite beta in which a portion of the framework silicon making up the zeolite has been substituted by another metal; and framework-substituted zeolite beta containing the aforementioned ammonium. As long as the framework structure of zeolite beta is present, zeolite beta having all of the aforementioned function and activity that makes up the subject refolding agent is basically not necessarily restricted to those provided as examples hereinabove.

The aforementioned function and activity of the subject refolding agent are expressed by bringing inactive or denatured protein into contact with the refolding agent, that is, by adsorption and desorption. During this process, the affinity between the surface of the refolding agent and the target protein is important, and in addition to this it is frequently also the case that protein adsorption/desorption is influenced by the dispersing solvent therefor, the denaturant, surfactant, and refolding factor in the dispersing solvent, the pH of the dispersing solvent, and so forth. As a consequence, the refolding activity of the refolding agent with respect to the target protein and the composition of the solution containing the target protein frequently varies among the different zeolite betas described above that can make up the refolding agent. As a rule, however, refolding agent comprising zeolite beta that contains an ammonium species has a higher refolding activity than in the absence of the ammonium species, and for this reason the use of refolding agent comprising zeolite beta containing an ammonium species and the use of refolding agent comprising framework-substituted zeolite beta containing an ammonium species is frequently preferred.

The ammonium species that should be present in the zeolite beta that makes up the subject refolding agent can be an ammonium species that tends to remain in the cavities present in the zeolite, for example, the ammonium ion; mono-, di-, tri-, and tetraalkylammonium ions where the alkyl is methyl, ethyl, propyl, butyl, and so forth; the ammonium ions of 5-, 6-, and 7-member cyclic aliphatic and aromatic amines and more particularly the piperidium ion, alkylpiperidium ion, pyridinium ion, alkylpyridinium ion, aniline ion, and N-alkylaniline ion; and formamide, acetamide, and their N-alkyl substitution products as examples of acid amides. However, basically any ammonium species that can enter the pores present in the zeolite beta can be used and there is no restriction to the ammonium species provided as examples hereabove.

The elements forming the framework of the zeolite beta that makes up the subject refolding agent are generally silicon and oxygen or silicon, oxygen, and aluminum; however, zeolite beta in which a portion of the silicon or aluminum has been substituted by another element and substituted zeolite beta containing the aforementioned ammonium species in the pores thereof can also provide refolding agent that effects function activation on an inactive protein. Typical examples of elements that can substitute for the framework silicon in zeolite beta are aluminum, boron, phosphorus, gallium, tin, titanium, iron, cobalt, copper, nickel, zinc, chromium, and vanadium, but there is no limitation to the preceding and basically any element that does not destroy the zeolite beta structure can be used. With regard to the amount of substitution, any amount of substitution that does not destroy the zeolite beta structure is unproblematic and the subject substituted zeolite beta will have the same ability to provide a refolding agent for inactive or denatured protein. With regard to other features of the present invention, the items described for the first aspect of the present invention are also similarly applied to the present invention.

A third aspect of the present invention is described in additional detail below.

The third aspect of the present invention comprises the following technical means.
(1) A protein refolding agent that has a protein refolding action that modulates the higher order structure of and activates a protein that is inactive due to a disordered higher order structure, the protein refolding agent comprising zeolite with the BEA structure (zeolite beta).
(2) The refolding agent according to (1), that carries out protein refolding in the presence of a protein denaturant, a surfactant, and/or a refolding buffer.
(3) The refolding agent according to (1), wherein the protein that is inactive due to a disordered higher order structure is a protein that is produced by an Escherichia coli expression system.
(4) The refolding agent according to (1), wherein the protein that is inactive due to a disordered higher order structure is a protein that is deactivated due to its thermal history.
(5) The refolding agent according to (1), wherein the zeolite beta contains ammonium ion, an organic ammonium ion, and/or urea.
(6) The refolding agent according to (5), wherein the organic ammonium ion is a mono-, di-, tri-, and/or tetraalkylammonium ion (where the alkyl group is methyl, ethyl, propyl, or butyl).
(7) The refolding agent according to (1), wherein the framework structure of the zeolite beta comprises oxygen and at least one element other than oxygen.
(8) The refolding agent according to (7), wherein the framework structure of the zeolite beta comprises silicon and oxygen or silicon, aluminum, and oxygen.
(9) The refolding agent according to any of (1) to (8), that manifests a protein refolding action through contact with a protein dispersed in a solution.
(10) The refolding agent according to any of (1) to (9), that causes refolding of the protein by a procedure in which the protein in a solution is adsorbed by mixing with the refolding agent or by introduction onto a column packed with the refolding agent and thereafter is desorbed.

Protein that may be processed by the refolding agent according to the present invention comprising zeolite beta is any inactive protein with an irregular higher order structure, but in particular will be conformation-disordered protein produced by, for example, an Escherichia coli expression system and known as an inclusion body, or protein deactivated for some reason, such as the thermal history. The refolding agent according to the present invention effects activation or generation of a native function of a protein by refolding the conformation of the protein by a process in which the protein is adsorbed to and desorbed from the refolding agent. However, the subject capability of the refolding agent is not necessarily limited to the preceding and is generally manifested by the following protocol. In other words, activation of a function of an inactive protein is carried out by the following protocol. That is, this protocol is carried out by a sequence in which the protein is first dissolved and dispersed in a solution containing denaturant and/or detergent and so forth; this is mixed with a solution containing the refolding agent according to the present invention, or is introduced onto a column packed with the refolding agent, in order to adsorb the protein to the refolding agent; and the protein is then desorbed from the refolding agent.

Displacement adsorption is generally used for desorption of the protein; however, there are no particular limitations here as basically any procedure can be used that does not impair activation of the function after desorption of the protein. Thus, changes in the pH or temperature can also be used, and these can also be used in combination with displacement adsorption. A salt as heretofore used for elution in column chromatography, such as sodium dodecyl sulfate or an alkali halide, is often used as the substance that induces desorption of the protein during displacement adsorption, and their co-use frequently also provides remarkable results. Accordingly, it is also possible during execution of protein desorption by displacement desorption to use the combination of various salts, such as those used for elution in column chromatography, with surfactant and/or refolding factor. These salts used in combination are not limited to those provided as examples here, and any salt can be used as long as it does not impair the activation of function after desorption of the protein.

Various supplementary procedures can also be carried out in combination with the aforementioned protocol in order to induce adsorption of the protein to the refolding agent according to the present invention or desorption therefrom. A typical example of such a procedure involves, for example, exposure to ultrasound or microwaves and/or application of a magnetic or electrical field. The protein refolding activity of the refolding agent is strongly manifested through the procedures and protocol as described above, causing the refolding of protein produced using, e.g., an Escherichia coli expression system, that has an as yet unformed higher order structure, or causing the refolding of protein deactivated for some reason, and thereby rapidly activating a native function that the protein should have. With regard to other features of the present invention, the items described for the first aspect of the present invention are also similarly applied to the present invention.

A fourth aspect of the present invention is described in additional detail below.

The fourth aspect of the present invention comprises the following technical means.
(1) A refolding molding comprising a molding that contains zeolite with the BEA structure (known as zeolite beta) that has the capacity, denoted as a refolding activity, to modulate and activate the higher order structure of a protein that is inactive due to a disordered higher order structure.
(2) The refolding molding according to (1), wherein the molding comprises zeolite beta or zeolite beta and a substrate that supports the zeolite beta.
(3) The refolding molding according to (1), that manifests a refolding activity upon contact with a protein.
(4) The refolding molding according to (1), that carries out the refolding of a protein in the presence of a protein denaturant, a surfactant, and/or a refolding buffer.
(5) The refolding molding according to (1), wherein the protein that is inactive due to a disordered higher order structure is a protein that is produced by an Escherichia coli expression system.
(6) The refolding molding according to (1), wherein the protein that is inactive due to a disordered higher order structure is a protein deactivated due to its thermal history.
(7) The refolding molding according to (1), wherein the zeolite beta contains ammonium ion and/or organic ammonium ion.
(8) The refolding molding according to (7), wherein the organic ammonium is a mono-, di-, tri-, and/or tetraalkylammonium ion (where the alkyl group is methyl, ethyl, propyl, or butyl).
(9) The refolding molding according to (1), wherein the framework structure of the zeolite beta comprises oxygen and at least one element other than oxygen.
(10) The refolding molding according to (9), wherein the framework structure of the zeolite beta comprises silicon and oxygen or silicon, aluminum, and oxygen.
(11) The refolding molding according to any of (1) to (10), that manifests a protein refolding activity through contact with a protein dispersed in a solution.
(12) The refolding molding according to any of (1) to (11), that has a function that causes refolding of the protein by a procedure in which the protein is adsorbed to the molding by mixing the protein in a solution with the refolding molding or by flowing or dripping the protein in a solution onto the molding and thereafter is desorbed.

The refolding molding according to the present invention comprises just zeolite with the BEA structure, so-called zeolite beta, or comprises zeolite beta and a substrate (support) that supports the zeolite beta. A support is absent in the former case, while in the latter case a support is attached. As a general matter, the substances known as zeolites are often difficult to mold by themselves due to their poor self-sinterability. As a consequence, insofar as concerns the fabrication of such moldings, that is, the design and control of their shape and configuration, the latter case, because it enables immobilization and/or coating of the zeolite beta on a support with a pre-arranged shape, in general frequently provides greater latitude and is more advantageous than the former case.

However, the method of fabrication, which begins with the decision on whether to use a support for the molding and includes the design and control of the shape and configuration, varies as a function of how the molding will be utilized and the configuration of use and thus is selected as appropriate. Accordingly, the known methods are all usable for fabrication of the molding under consideration and may be selected as appropriate, and combinations of these methods can also be used, and as a consequence there are no particular restrictions on fabrication of this molding and in particular a detailed description or discussion is unnecessary. This notwithstanding, several examples will be provided hereinbelow of methods for fabricating the molding under consideration, that is, methods for designing and controlling the shape and configuration. Typical conventional methods for the fabrication of zeolite moldings, i.e., in situ zeolite synthesis, dry gel conversion, solid-phase conversion (refer to Stud. Surf. Sci. Catal. Vol. 125 (1999) 1-12; Hyomen [Surface], Vol. 37 (1999) 537-557), can also be used for fabrication of the molding under consideration, both for the support-free version and the support-attached version. Methods usable for the fabrication of support-attached moldings include incorporation into an organic polymer (Zeolites, Vol. 16 (1996) 70), bonding/molding of the zeolite beta by means of an inorganic powder such as alumina, and immobilization of the zeolite beta on the support by means of a water-insoluble adhesive, but there is no limitation to the preceding.

The shape and configuration of the molding under consideration is selected as appropriate from, for example, chip shapes, film or membrane shapes, pellet shapes, and bead shapes, in correspondence to how the molding will be utilized and the configuration of use. In the particular case of the support-attached moldings under consideration, the zeolite beta can be immobilized and/or coated by the aforementioned methods on supports with a variety of shapes, for example, plates, spheres, cylinders, tubes, columns, troughs, and U-shaped channels. This offers the advantage of enabling the molding to be executed in any desired shape. The support in this case can be exemplified by glass; quartz; various ceramics such as alumina, silica, cordierite, and mullite; cellulosics such as paper; and various organic polymers such as Teflon®, nylon, polyethylene, polypropylene, and polyethylene terephthalate (PET). However, basically any support is acceptable that is water insoluble and that does not negatively affect protein and the support is not limited to those provided above as examples.

Protein that may be processed by the refolding molding according to the present invention is any inactive protein with an irregular higher order structure, but in particular will be conformation-disordered protein produced by, for example, an Escherichia coli expression system and known as an inclusion body, or protein deactivated for some reason, such as the thermal history. The refolding molding according to the present invention effects activation or generation of a native function of a protein by refolding the conformation of the protein by a process in which the protein is adsorbed to and desorbed from the refolding molding. However, the subject capability of the refolding molding is not necessarily limited to the preceding and is generally manifested by the following protocol. In other words, activation of a function of an inactive protein is carried out by the following protocol. This protocol is carried out by a sequence in which the protein is first dissolved and dispersed in a solution containing denaturant and/or detergent and so forth; this solution is then mixed with the refolding molding according to the present invention or is poured onto, flowed into or over, or dripped onto the refolding molding, in order to adsorb the protein to the refolding molding; and the protein is then desorbed from the refolding molding. There is no specific requirement for a separator, such as a centrifugal separator, in these steps. With regard to other features of the present invention, the items described for the first aspect of the present invention are also similarly applied to the present invention.

The present invention relates, inter alia, to a method for activating a function of an inactive protein, and the following effects are achieved by the present invention:
1) a native function or activity of a protein produced by, for example, an Escherichia coli expression system and being inactive due to an as yet unformed higher order structure, or of a protein deactivated due to a change in its conformation for some reason, can be activated by refolding;
2) this method is useful for the highly efficient refolding of inclusion bodies;
3) an efficient method is provided that has a high refolding rate, that is versatile and generalizable, and that is applicable to a variety of proteins;
4) the zeolite beta comprising the function activator used by the present invention is inexpensive and can be used repeatedly;
5) this method can be applied to the refolding of a variety of conformation-disordered proteins, including large proteins with molecular weights in excess of 100,000; and
6) the combination of the method according to the present invention with, for example, a protein synthesis process based on an Escherichia coli expression system, enables the elaboration of a novel process for manufacturing active protein that produces protein having a controlled higher order structure and a native function inherent to the protein.

The invention additionally relates to a reagent kit that is used in the procedures and/or processes for activation of a function of an inactive protein and further relates to a method of using this reagent kit. The following separate effects are achieved by the present invention:
1) an all-purpose reagent set, that is, an all-purpose kit, can be selected that can activate a native function of a wide range of inactive proteins, regardless of the type of protein;
2) the use of this kit to treat such protein, for example, a protein produced by, for example, an Escherichia coli expression system and being inactive due to an as yet unformed higher order structure, or a protein deactivated due to a change in its conformation for some reason, enables the activation of a native function or activity of the protein by refolding;
3) the subject kit is also effective on the protein in inclusion bodies and is useful in providing an efficient method for the refolding of inclusion bodies utilizing this activity;
4) the method for activating the function of inactive protein through the use of this kit provides a versatile, generalizable, and efficient method that has a high refolding rate and that can be applied to a variety of denatured proteins regardless of the chain length and sequence of the amino acids making up the protein;
5) the zeolite with the BEA structure, that is, zeolite beta, that makes up the refolding agent that is an essential component of this kit is inexpensive and can be used repeatedly;
6) the method for activating the function of inactive protein utilizing the subject kit can be applied to the refolding of a variety of conformation-disordered proteins, including large proteins with molecular weights in excess of 100,000; and
7) the combination of the activation of a function of an inactive protein using the subject kit with, for example, a protein synthesis process based on an Escherichia coli expression system, enables the elaboration and establishment of a novel process for manufacturing active protein that produces protein that has a controlled higher order structure and that is provided with a native function inherent to the protein.

The invention additionally relates to a refolding agent and a molding that are a substance and material that have the capacity to activate a function of an inactive protein. The following separate effects are achieved by the present invention:
1) an all-purpose substance or material, that is, refolding agent comprising zeolite with the BEA structure (zeolite beta), can be selected that can activate a native function of a wide range of inactive proteins, regardless of the type of protein;
2) effecting contact between the refolding agent according to the present invention and such protein, for example, a protein produced by, for example, an Escherichia coli expression system and being inactive due to an as yet unformed higher order structure, or a protein deactivated due to a change in its conformation for some reason, enables the activation of a native function or activity of the protein by refolding;
3) the refolding agent according to the present invention is also effective on the protein in inclusion bodies and is useful in providing an efficient method for the refolding of inclusion bodies;
4) the method for activating the function of inactive protein through contact with the refolding agent according to the present invention is a versatile, generalizable, and efficient method that has a high refolding rate and that can be applied to a variety of denatured proteins regardless of the chain length and sequence of the amino acids making up the protein;
5) the zeolite beta that makes up the refolding agent according to the present invention is inexpensive and can be used repeatedly;
6) the method for activating the function of inactive protein utilizing the refolding agent according to the present invention can be applied to the refolding of a variety of conformation-disordered proteins, including large proteins with molecular weights in excess of 100,000; and
7) the combination of the activation of a function of an inactive protein by the refolding agent according to the present invention with, for example, a protein synthesis process based on an Escherichia coli expression system, enables the elaboration and establishment of a novel process for manufacturing active protein that produces protein that has a controlled higher order structure and that is provided with a native function inherent to the protein.

### BRIEF DESCRIPTION OF THE DRAWINGS

Figure 1 shows the results of an electrophoretic gel shift assay.
Figure 2 shows the recovery rate and activity recovery rate for refolded protein.
Figure 3 shows the results of an electrophoretic gel shift assay.

### BEST MODE FOR CARRYING OUT THE INVENTION

The invention is specifically described hereinbelow based on examples and comparative examples, but the invention is in no way limited by the examples and other material that follow.

### Examples and Comparative Examples for the First Aspect of the Present Invention

The examples provided below describe the activation of a function of protein produced by an Escherichia coli expression system and denatured protein; however, the present invention is neither limited to nor restricted by these examples.

### 1) Preparation of materials

### (a) The function activator

The following were used as the function activator:
uncalcined zeolite beta (Na-BEA) as shown in Table 1 below, synthetic zeolite beta as shown in Table 1, calcined zeolite beta obtained by calcination of the synthetic zeolite, and comparative products as shown in Table 1 for Comparative Examples 1 to 15.

### (b) The denatured protein solution

The proteins used are described under "protein" and "remarks" in Table 1 and included RPA70 (Drosophila melanogaster origin) and P53 (human origin).

### (c) The refolding buffer

The salt concentration of the refolding buffer was investigated using Na-BEA as the zeolite and RPA70 as the protein. The refolding buffer used 20 mM TrisHCl pH 7.5, 0.5 M NaCl, 20 mM 2-mercaptoethanol, 2.5% (w/v) polyethylene glycol 20,000, and nonionic detergent wherein 1% (v/v) Tween 20, Triton X-100, and NP-40 were used as the detergent. The details of the refolding buffers actually used in the examples and comparative examples are shown in Table 2 below.

### 2) The refolding protocol

100 mg function activator was introduced into a 1.5 mL Eppendorf tube followed by the addition of 0.5 mL 6 M guanidine hydrochloride, 20 mM trisaminomethane trihydrochloride (TrisHCl) pH 7.5, 0.5 M NaCl, and 20 mM 2-mercaptoethanol and suspension. To this was then added 6 M guanidine hydrochloride and 20 mM 2-mercaptoethanol followed by holding for one hour on ice, after which 0.5 mL of the denatured protein solution (concentration from 0.5 to 1.0 mg/mL) was added. In order to ensure adsorption of the protein on the function activator, this mixture was stirred for 1 hour with a Rotary Culture RCC-100 (Iwaki Glass Co., Ltd.) placed in a cold room.

The function activator was then sedimented by centrifugation for 5 seconds at 10000 × g and the supernatant was removed. In order to completely remove the protein denaturant from the sedimented function activator, it was washed 4 times with 1 mL 20 mM TrisHCl pH 7.5, 20 mM 2-mercaptoethanol followed by centrifugation for 5 seconds at 10000 × g and discard of the supernatant thereby produced. The remaining function activator was suspended by the addition thereto of 1 mL refolding buffer (comprising 50 mM HEPES pH 7.5, 0.5 M NaCl, 20 mM 2-mercaptoethanol, refolding factor, and nonionic detergent).

In order to desorb and elute the protein adsorbed on the function activator, this suspension was again stirred in the cold with the Rotary Culture RCC-100 (Iwaki Glass Co., Ltd.). The function activator was thereafter sedimented by centrifugation for 5 seconds at 10000 × g, and the protein-containing supernatant was transferred to a new Eppendorf tube and this was used for activity measurement (assay).

Methods appropriate to the action of the protein used were employed for the activity measurements. Specifically, the activity was measured using three types of measurements, i.e., a gel shift assay, a polymerase assay, and measurement of lysozyme activity.

### 3) Activity measurement protocols

### (a) The gel shift assay

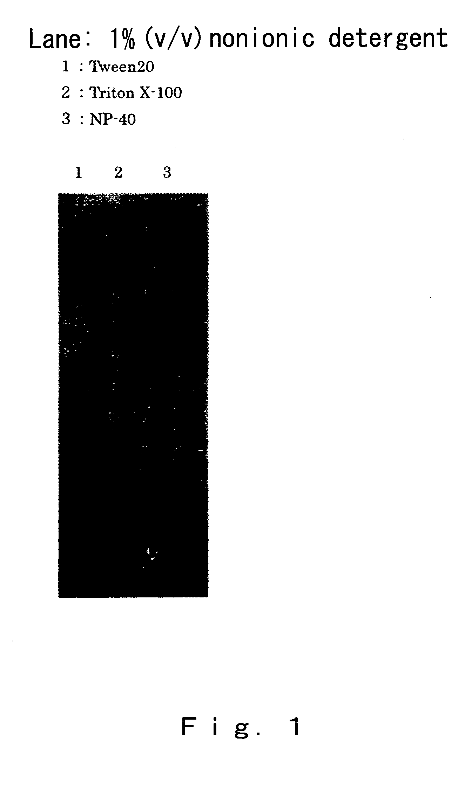

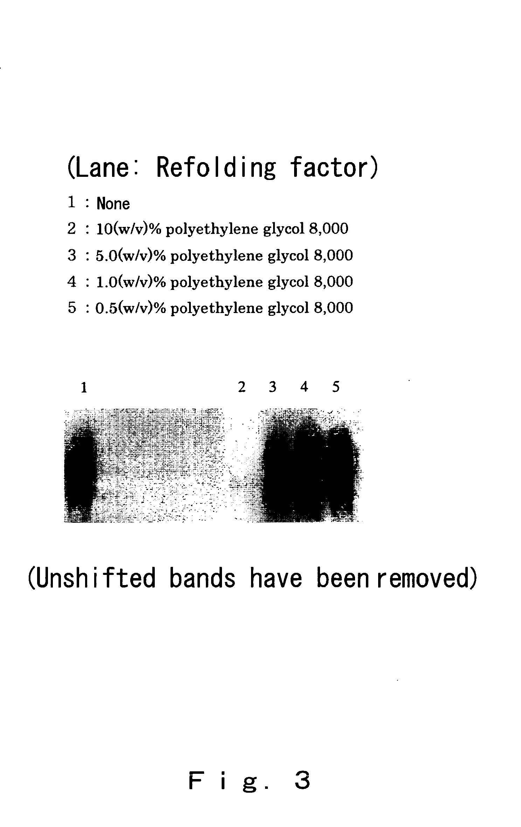

1 pmol radioisotope-labeled DNA oligonucleotide and the refolded protein were incubated for 30 minutes on ice in a solution with a composition of 25 mM HEPES pH 7.4, 50 mM KCl, 20% glycerol, 0.1% NP-40, 1 mM DTT, and 1 mg/mL bovine serum albumin. This was followed by electrophoresis at 4°C on 4.5% polyacrylamino gel using 0.5× TBE buffer. The results are shown in Figures 1 and 3.

When DNA binding to the protein was present (that is, when activity was present), the protein underwent binding to the DNA, which slowed down electrophoresis and caused band shifting, thereby enabling a determination of activity (that is, the refolding rate).

### (b) The polymerase assay

Poly(dA)oligo(dT)₁₂₋₁₈ or DNase I-activated calf thymus DNA was used as the template DNA. The reaction solution had a composition (final concentration) of 50 nmM TrisHCl pH 7.5, 1 mM DTT, 15% glycerol, 5 mM MgCl₂, 0.5 µM dTTP (cold) (thymidine triphosphate), and [³H]-dTTP (5 mCi/mL:100-500 cpm/pmol). The protein (enzyme) sample solution was first added to and suspended in 10 µL reaction solution that was twice as concentrated as that given above followed by incubation for 1 hour at 37°C, after which the reaction was stopped by holding on ice.

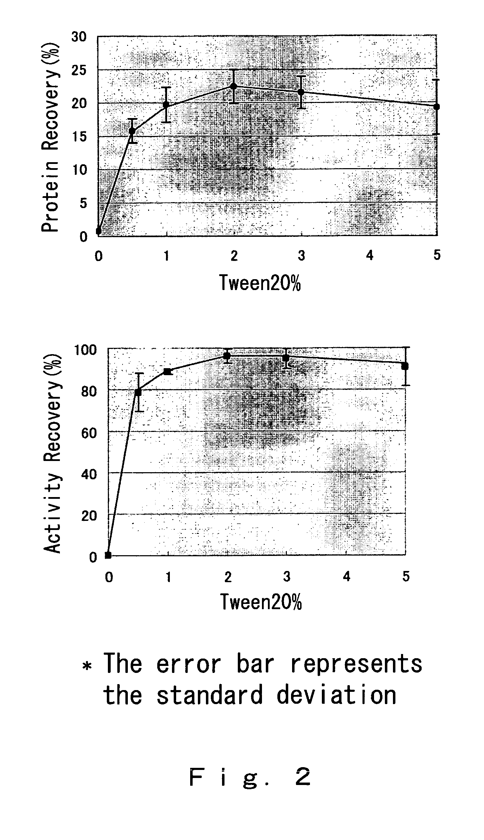

The reaction solution was then dripped onto DE81 paper that had been cut into a square. After drying, this was transferred to a beaker and was washed in order to dissolve and remove the unreacted dTTP. This wash consisted of first 3X with 5% aqueous disodium hydrogen phosphate solution, then 3X with distilled water, then 2X with ethanol, and was followed by drying. The dry DE81 paper obtained in this manner was placed in a scintillator-containing vial and the radioactivity (cpm) was measured with a scintillation counter. Stronger activity by the enzyme sample resulted in greater incorporation of radioisotope-labeled dTTP in the thereby synthesized DNA and thus in greater radioactivity, and the protein activity was determined on this basis. The refolded protein recovery rate and the activity recovery rate are shown in Figure 2 for the use of Tween 20.

### (c) Measurement of lysozyme activity

The bacteria M. lysodeikticus was selected as the substrate and was suspended in 50 mM phosphate buffer to prepare a substrate solution with a concentration of 0.16 mg/mL. 20 µL of the protein (lysozyme enzyme) solution was added to 480 µL of this substrate solution followed by incubation for 30 minutes at room temperature. This was followed by measurement of the absorbance at a wavelength of 450 nm.

Lysozyme has the capacity to degrade the cell wall of bacteria, and as a result the higher this capacity, that is, the activity, the greater the decline in absorbance. 1 unit of lysozyme activity was defined as a decline in absorbance at 450 nm of 0.001 per minute.

The activity (refolding rate) and protein recovery rate, which are the results for the subject examples obtained by the procedures and protocols described above, are shown in Table 1 in combination with the results for the comparative examples. The refolding buffers used in the examples and comparative examples are shown in Table 2. As shown by the examples, activity native to the proteins, for example, DNA binding activity, is produced by refolding. The present invention is useful as a highly versatile, highly generalizable refolding method that is applicable to a variety of denatured and deactivated proteins and proteins that have an as yet unformed higher order structure; however, the application of the present invention is not limited to the proteins shown in the examples and the present invention can be applied to any protein.

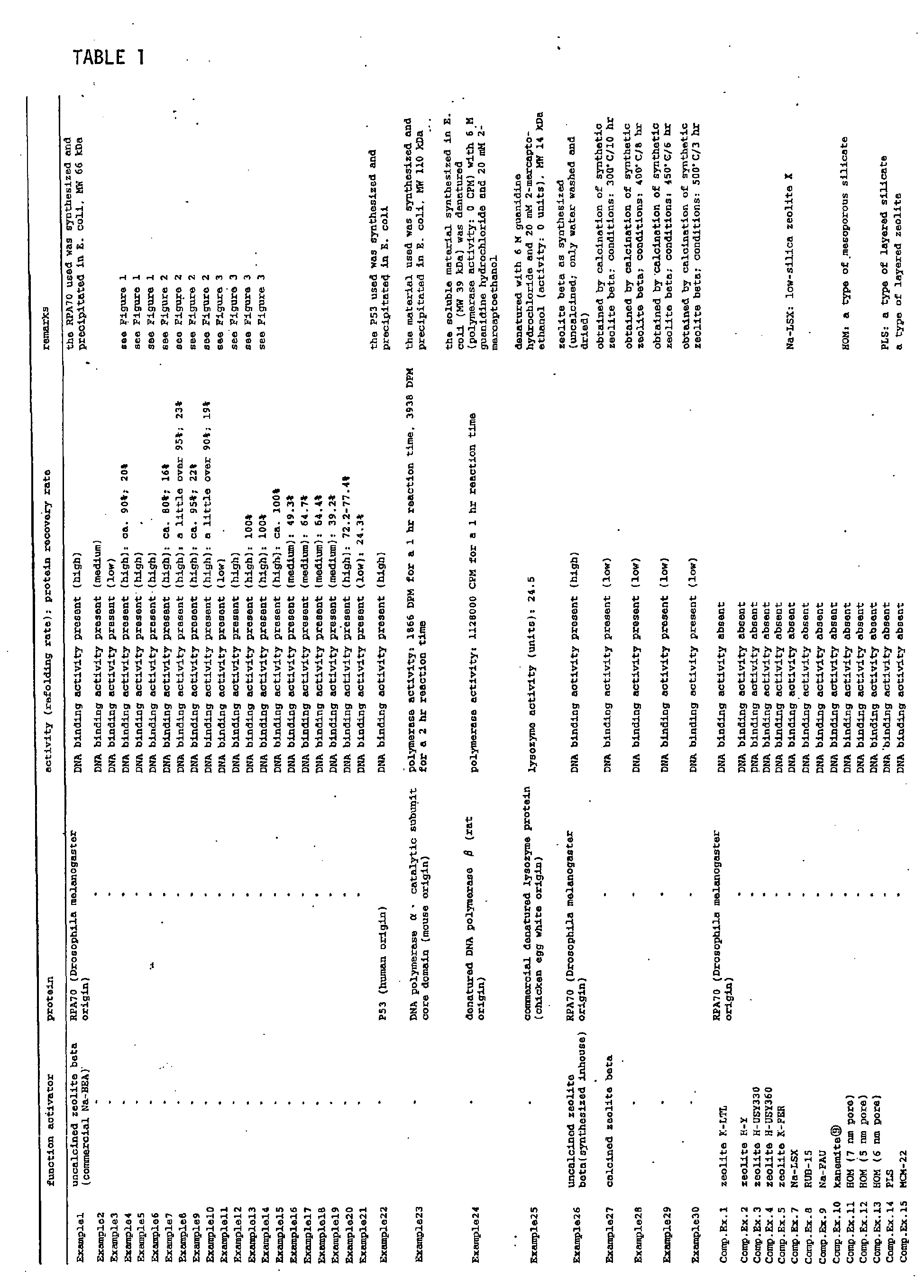

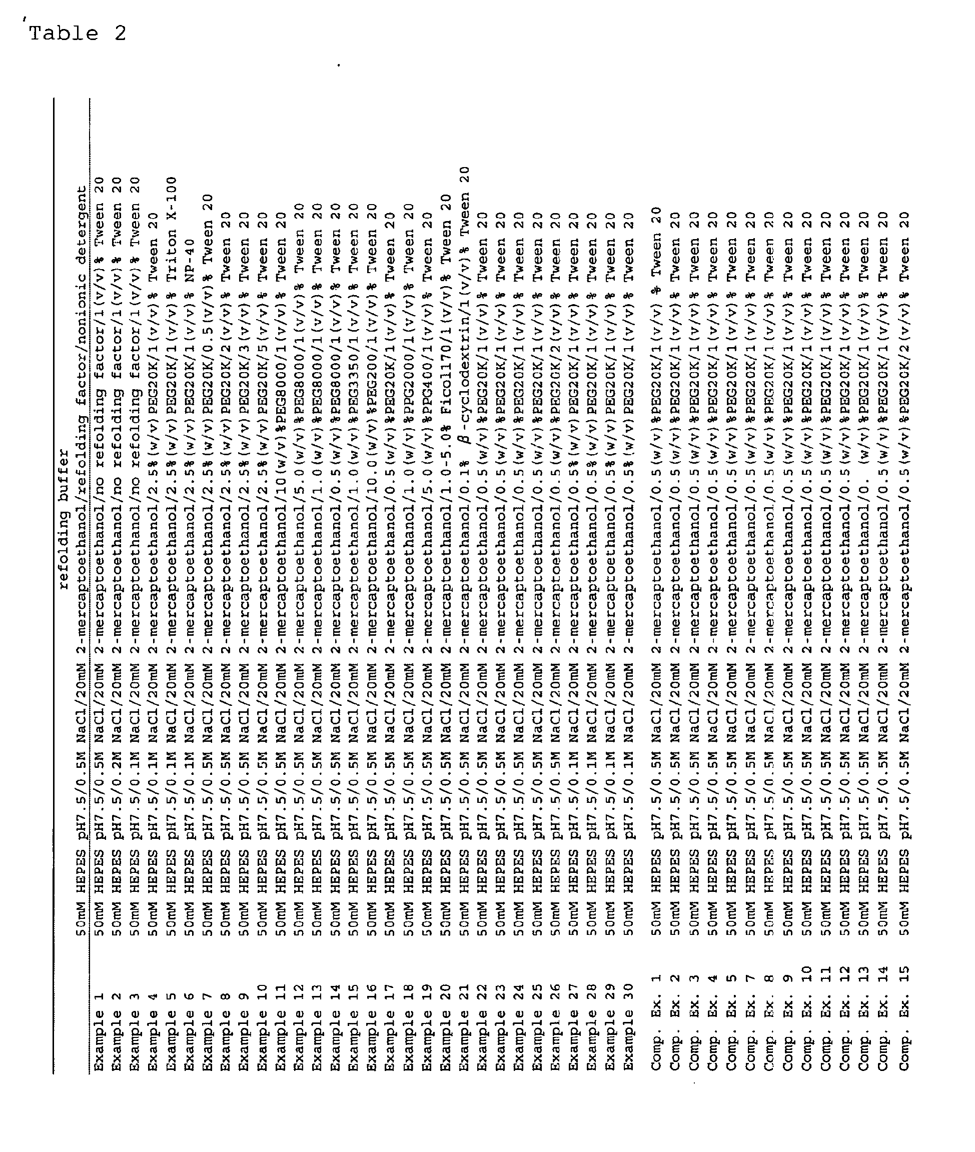

### Examples and Comparative Examples for the Second Aspect of the Present Invention

These examples provided below describe the activation of a function of protein produced by an Escherichia coli expression system and denatured protein; however, the present invention is neither limited to nor restricted by these examples.

### 1) Preparation of materials

### (a) The refolding agent

The following were used as the refolding agent (function activator) according to the present invention, as shown in Tables 3 and 4 below: commercially available zeolite beta, synthetic zeolite beta, calcined zeolite beta obtained by calcination of the preceding, zeolite beta containing various ammonium species, framework-substituted zeolite beta, and framework-substituted zeolite beta containing various ammonium species. The substances listed for the comparative examples in Table 5 were used as comparative products. These substances were not zeolites with the beta structure. For example, the zeolites in Comparative Examples 19 and 20 were silica and silica · alumina in which the BEA structure had yet to form due to the use of an inadequate synthesis time and were mainly amorphous structures.

### (b) The denatured protein solutions

The proteins used as the target protein for activation are described under "protein" and "remarks" in Tables 3 to 5 and included RP70 (Drosophila melanogaster origin) and P53 (human origin).

### (c) The refolding buffer

A solution with the composition 50 mM HEPES pH 7.5/0.5 M NaCl/20 mM 2-mercaptoethanol/2.5% (w/v) polyethylene glycol 20000 (refolding factor)/1% (v/v) Tween 20 (detergent) was generally used as the refolding buffer. The details of the refolding buffers used are shown in Tables 6 to 8.

### 2) The refolding protocol

100 mg function activator was introduced into a 1.5 mL Eppendorf tube followed by the addition of 0.5 mL 6 M guanidine hydrochloride · 20 mM trisaminomethane trihydrochloride (TrisHCl) pH 7.5 · 0.5 M NaCl · 20 mM 2-mercaptoethanol and suspension. To this was then added 6 M guanidine hydrochloride · 20 mM 2-mercaptoethanol followed by holding for one hour on ice, after which 0.5 mL of the denatured protein solution (concentration from 0.5 to 1.0 mg/mL) was added. In order to ensure adsorption of the protein on the function activator, this mixture was stirred for 1 hour with a Rotary Culture RCC-100 (Iwaki Glass Co., Ltd.) placed in a cold room.

The function activator was then sedimented by centrifugation for 5 seconds at 10000 × g and the supernatant was removed. In order to completely remove the protein denaturant from the sedimented function activator, it was washed 4 times with 1 mL 20 mM TrisHCl pH 7.5 · 20 mM 2-mercaptoethanol followed by centrifugation for 5 seconds at 10000 × g and discard of the supernatant thereby produced. The remaining function activator was suspended by the addition thereto of 1 mL refolding buffer (comprising 50 mM HEPES pH 7.5, 0.5 M NaCl, 20 mM 2-mercaptoethanol, refolding factor, and nonionic detergent).

In order to desorb and elute the protein adsorbed on the function activator, this suspension was again stirred in the cold with the Rotary Culture RCC-100 (Iwaki Glass Co., Ltd.). The function activator was thereafter sedimented by centrifugation for 5 seconds at 10000 × g, and the protein-containing supernatant was transferred to a new Eppendorf tube and this was used for activity measurement (assay).

Methods appropriate to the action of the protein used were employed for the activity measurements. Specifically, the activity was measured using four types of measurements, i.e., a gel shift assay, a polymerase assay, lysozyme activity measurement, and measurement of the topoisomerase I activity.

### 3) Activity measurement protocols

### (a) The gel shift assay

1 pmol radioisotope-labeled DNA oligonucleotide and the refolded protein were incubated for 30 minutes on ice in a solution with a composition of 25 mM HEPES pH 7.4 · 50 mM KCl · 20% glycerol · 0.1% NP-40 · 1 mM DTT · 1 mg/mL bovine serum albumin. This was followed by electrophoresis at 4°C on 4.5% polyacrylamino gel using 0.5× TBE buffer.

When DNA binding to the protein was present (that is, when activity was present), the protein underwent binding to the DNA, which slowed down electrophoresis and caused band shifting, thereby enabling a determination of activity (that is, the refolding rate).

### (b) The polymerase assay

Poly (dA) oligo (dT)₁₂₋₁₈ or DNase I-activated calf thymus DNA was used as the template DNA. The reaction solution had a composition (final concentration) of 50 nmM TrisHCl pH 7.5 · 1 mM DTT · 15% glycerol · 5 mM MgCl₂ · 0.5 µM dTTP (cold) (thymidine triphosphate) · [3H]-dTTP (5 mCi/mL:100-500 cpm/pmol). The protein (enzyme) sample solution was first added to and suspended in 10 µL reaction solution that was twice as concentrated as that given above followed by incubation for 1 hour at 37°C, after which the reaction was stopped by holding on ice.

The reaction solution was then dripped onto DE81 paper that had been cut into a square. After drying, this was transferred to a beaker and was washed in order to dissolve and remove the unreacted dTTP. This wash consisted of first 3X with 5% aqueous disodium hydrogen phosphate solution, then 3X with distilled water, then 2X with ethanol, and was followed by drying. The dry DE81 paper obtained in this manner was placed in a scintillator-containing vial and the radioactivity (cpm) was measured with a scintillation counter. Stronger activity by the enzyme sample resulted in greater incorporation of radioisotope-labeled dTTP in the thereby synthesized DNA and thus in greater radioactivity, and the protein activity was determined on this basis.

### (c) Measurement of lysozyme activity

The bacteria M. lysodeikticus was selected as the substrate and was suspended in 50 mM phosphate buffer to prepare a substrate solution with a concentration of 0.16 mg/mL. 20 µL of the protein (lysozyme enzyme) solution was added to 480 µL of this substrate solution followed by incubation for 30 minutes at room temperature. This was followed by measurement of the absorbance at a wavelength of 450 nm.

Lysozyme has the capacity to degrade the cell wall of bacteria, and as a result the higher this capacity, that is, the activity, the greater the decline in absorbance. 1 unit of lysozyme activity was defined as a decline in absorbance at 450 nm of 0.001 per minute.

### (d) Measurement of the topoisomerase I activity

0.5 µg supercoiled pBR322 and the topoisomerase I protein were suspended in reaction buffer (10 mM TrisHCl , pH 7.5, 150 mM NaCl, 5 mM beta-mercaptoethanol, 0.5 mM EDTA). After incubation for 30 minutes at 37°C, 0.1% SDS was added to stop the reaction. 0.5 µg/mL proteinase K was then added followed by incubation for 30 minutes at 37°C in order to degrade the topoisomerase I protein in the solution. The solution was subsequently subjected to electrophoresis on 1% (w/v) agarose and the DNA was stained with 0.5 µg/mL ethidium bromide. The topoisomerase I activity was measured based on observation of an upward shifted DNA band with a UV trans illuminator.

The activity (refolding rate) and protein recovery rate, which are the results for the subject examples obtained by the procedures and protocols described above, are shown in Tables 3 and 4 while the results for the comparative examples are shown in Table 5. As shown by the examples, activity native to the proteins, for example, DNA binding activity, is produced by refolding. The zeolite beta according to the present invention is useful as a highly versatile, highly generalizable refolding agent that is applicable to a variety of denatured and deactivated proteins and proteins that have an as yet unformed higher order structure; however, the application thereof is not limited to the proteins shown in the examples and the zeolite beta according to the present invention can be applied to any protein.

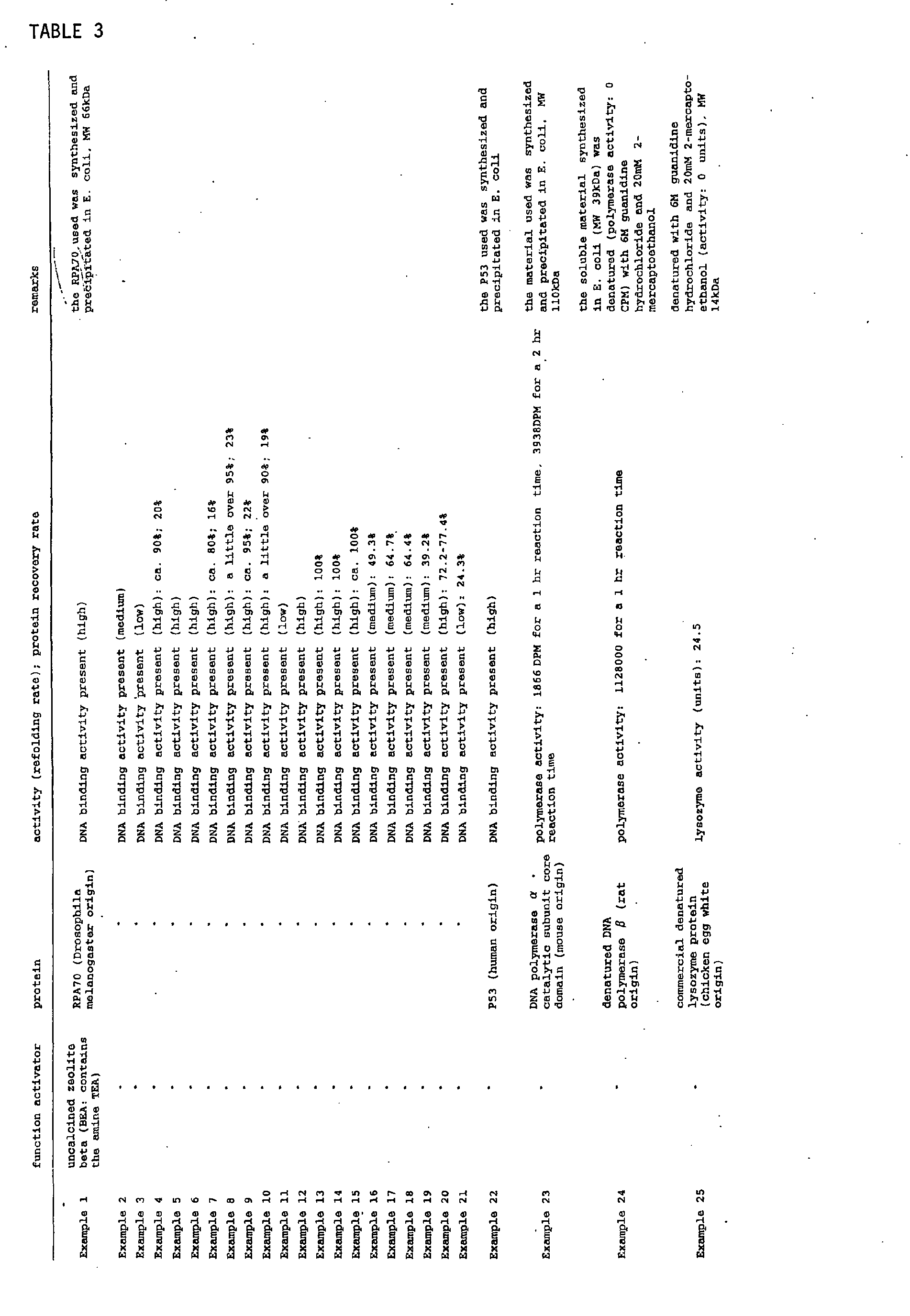

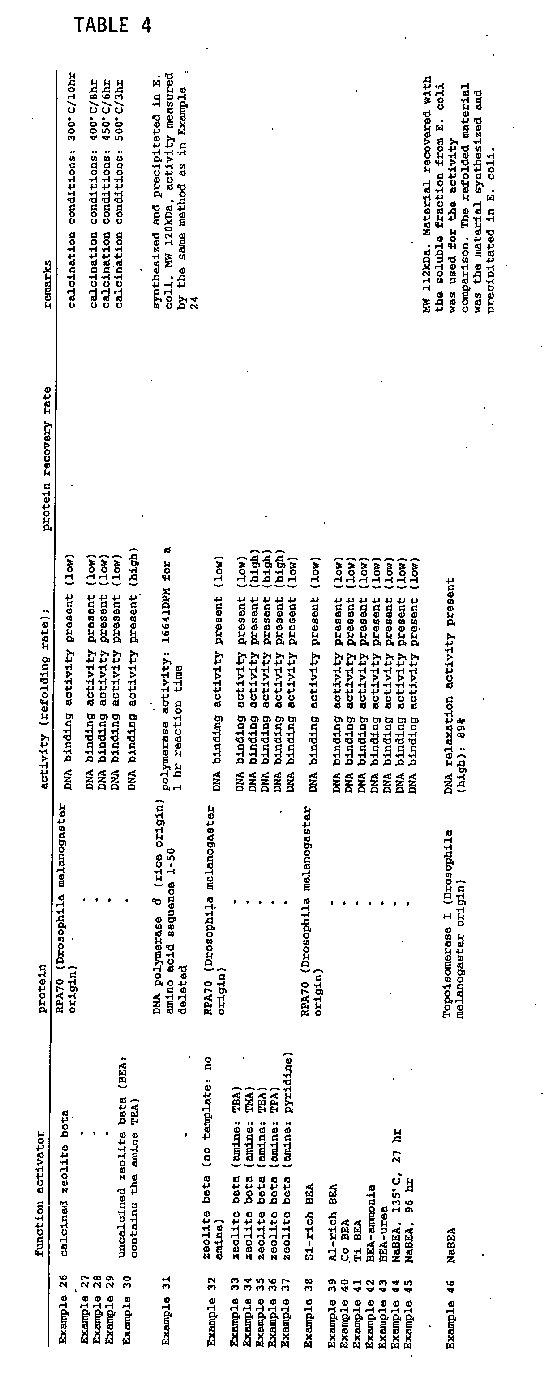

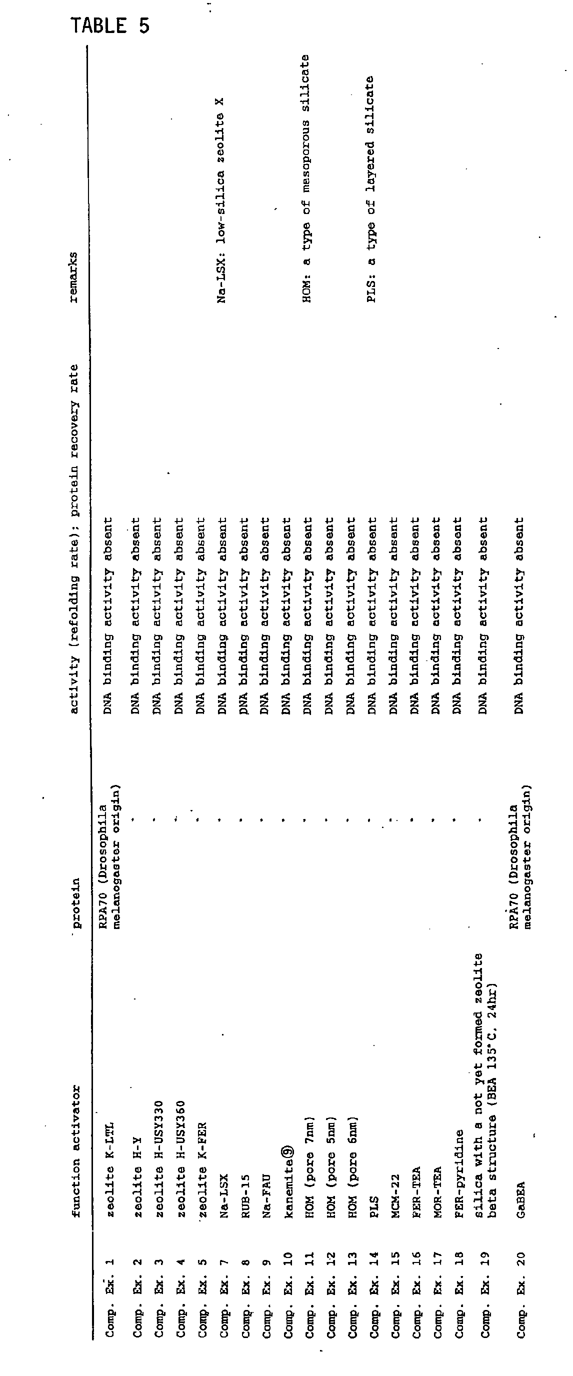

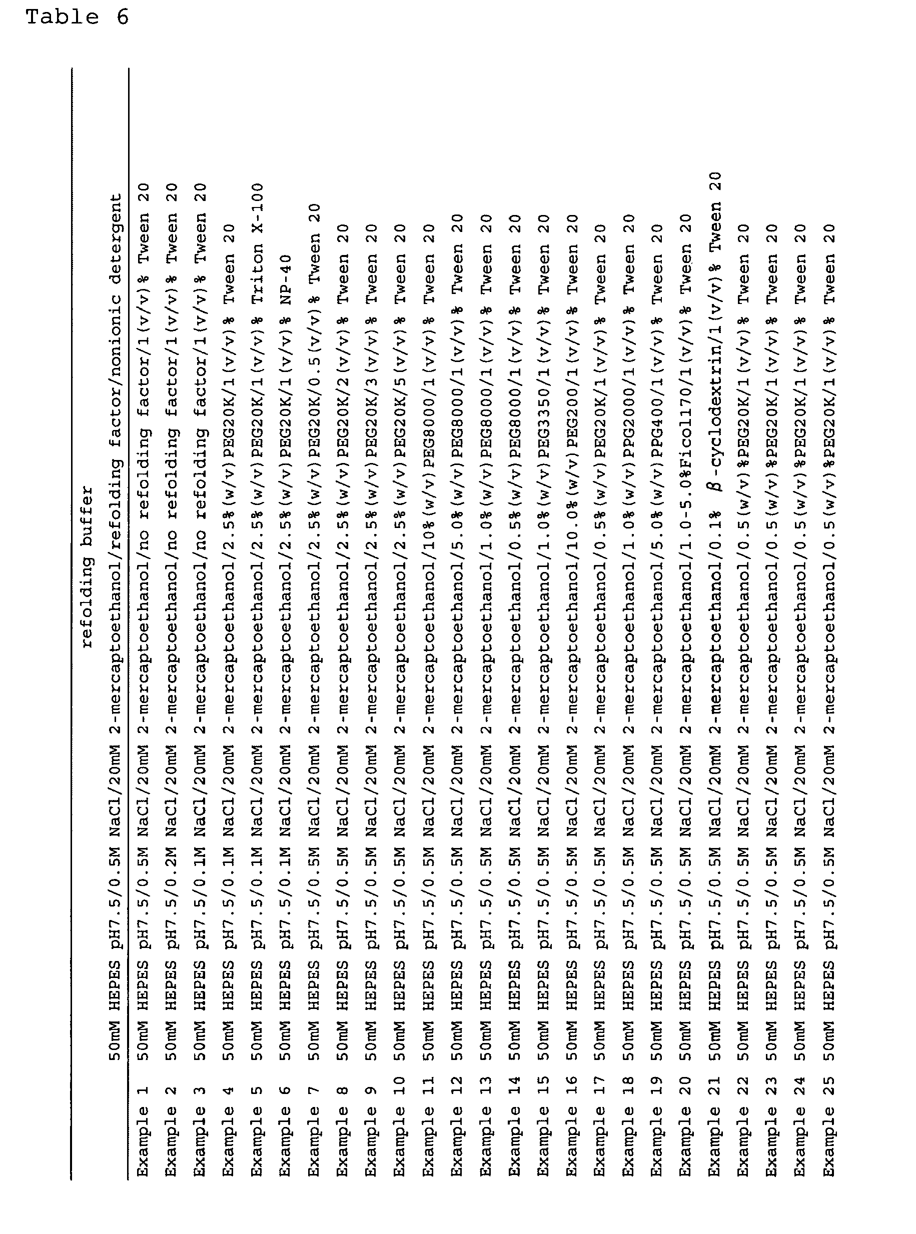

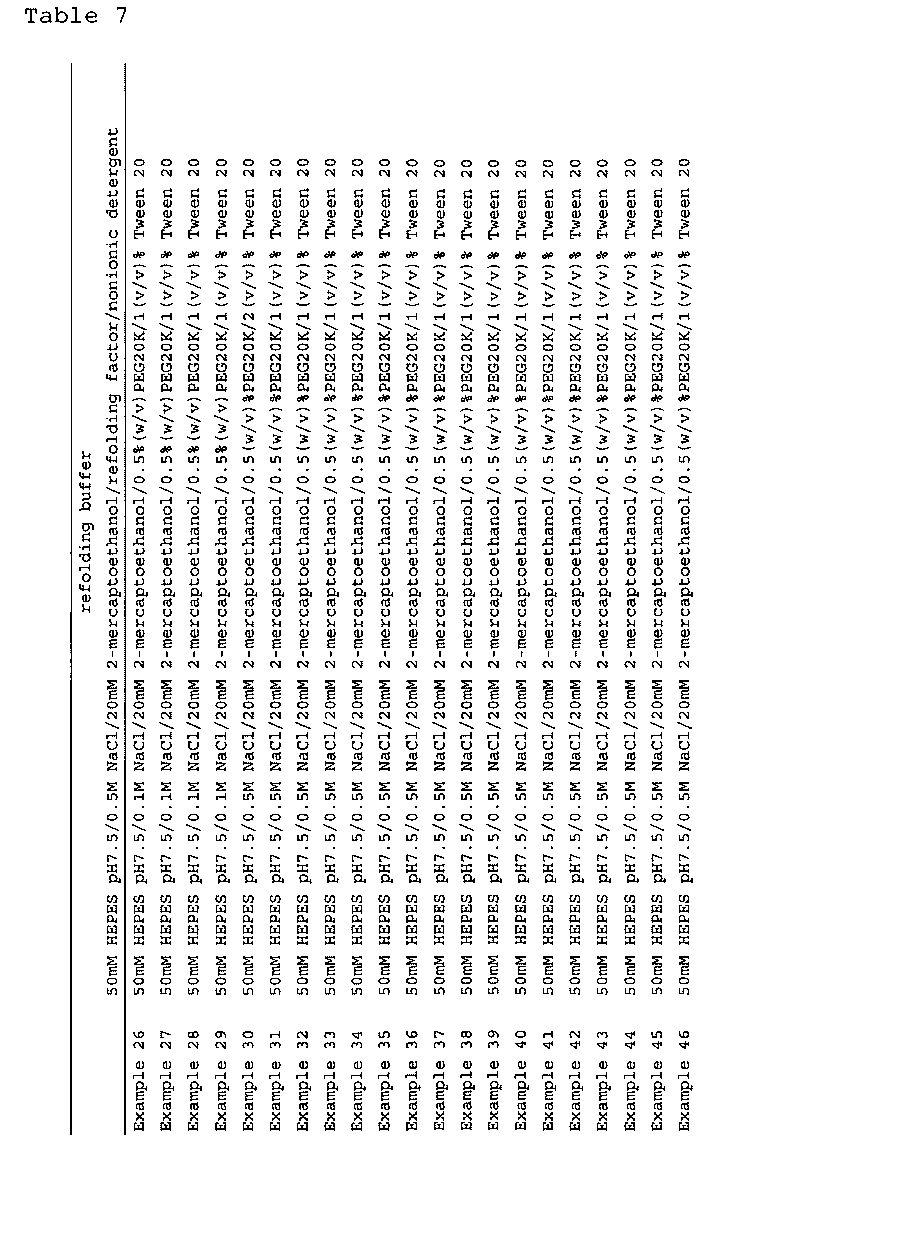

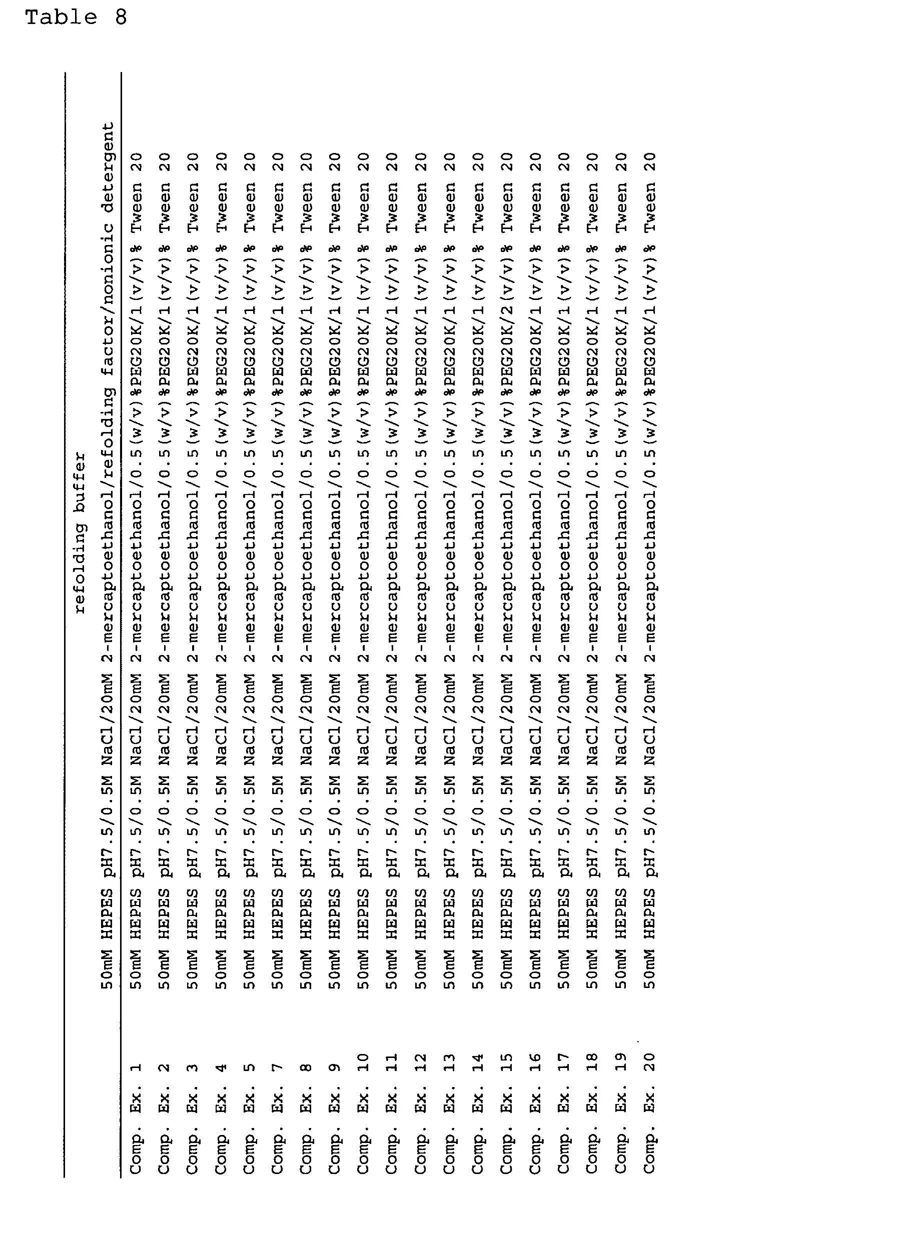

### Examples and Comparative Examples for the Third Aspect of the Present Invention

These examples provided below describe the activation of a function of protein produced by an Escherichia coli expression system and denatured protein; however, the present invention is neither limited to nor restricted by these examples.

### 1) Preparation of materials

### (a) The refolding agent

The following were used as the refolding agent (function activator), as shown in Tables 9 and 10 below: commercially available zeolite beta, synthetic zeolite beta, calcined zeolite beta obtained by calcination of the preceding, zeolite beta containing various ammonium species, framework-substituted zeolite beta, and framework-substituted zeolite beta containing various ammonium species. The substances listed for the comparative examples in Table 13 were used as comparative products. These substances were not zeolites with the beta structure. For example, the substances in Comparative Examples 19 and 20 were silica and silica alumina in which the BEA structure had yet to form due to the use of an inadequate synthesis time and were mainly amorphous structures.

### (b) The denatured protein solutions

The proteins described in Tables 9 and 10, RP70 (Drosophila melanogaster origin), P53 (human origin), and so forth were used as the target proteins for activation.

### (c) The refolding buffer

A solution with the composition 50 mM HEPES pH 7.5/0.5 M NaCl/20 mM 2-mercaptoethanol/2.5% (w/v) polyethylene glycol 20000 (refolding factor)/1% (v/v) Tween 20 (detergent) was generally used as the refolding buffer. The details of the refolding buffers used are shown in Tables 11, 12, and 14.

### 2) The refolding protocol

100 mg refolding agent was introduced into a 1.5 mL Eppendorf tube followed by the addition of 0.5 mL 6 M guanidine hydrochloride · 20 mM trisaminomethane trihydrochloride (TrisHCl) pH 7.5 · 0.5 M NaCl · 20 mM 2-mercaptoethanol and suspension. To this was then added 6 M guanidine hydrochloride · 20 mM 2-mercaptoethanol followed by holding for one hour on ice, after which 0.5 mL of the denatured protein solution (concentration from 0.5 to 1.0 mg/mL) was added. In order to ensure adsorption of the protein on the refolding agent, this mixture was stirred for 1 hour with a rotary culture unit (Rotary Culture RCC-100 from Iwaki Glass Co., Ltd.) placed in a cold room.

The refolding agent was then sedimented by centrifugation for 5 seconds at 10000 × g and the supernatant was removed. In order to completely remove the protein denaturant from the sedimented refolding agent, it was washed 4 times with 1 mL 20 mM TrisHCl pH 7.5 · 20 mM 2-mercaptoethanol followed by centrifugation for 5 seconds at 10000 × g and discard of the supernatant thereby produced. The remaining refolding agent was suspended by the addition thereto of 1 mL refolding buffer (comprising 50 mM HEPES pH 7.5, 0.5 M NaCl, 20 mM 2-mercaptoethanol, refolding factor, and nonionic detergent).

In order to desorb and elute the protein adsorbed on the refolding agent, this suspension was again stirred in the cold with a rotary culture unit (Rotary Culture RCC-100, Iwaki Glass Co., Ltd.). The refolding agent was thereafter sedimented by centrifugation for 5 seconds at 10000 × g, and the protein-containing supernatant was transferred to a new Eppendorf tube and this was used for activity measurement (assay).

Methods appropriate to the action of the protein used were employed for the activity measurements. Specifically, the activity was measured using four types of measurements, i.e., a gel shift assay, a polymerase assay, lysozyme activity measurement, and measurement of the topoisomerase I activity.

### 3) Activity measurement protocols

### (a) The gel shift assay

1 pmol radioisotope-labeled DNA oligonucleotide and the refolded protein were incubated for 30 minutes on ice in a solution with a composition of 25 mM HEPES pH 7.4 · 50 mM KCl · 20% glycerol · 0.1% NP-40 · 1 mM DTT · 1 mg/mL bovine serum albumin. This was followed by electrophoresis at 4°C on 4.5% polyacrylamino gel using 0.5× TBE buffer.

When DNA binding to the protein was present (that is, when activity was present), the protein underwent binding to the DNA, which slowed down electrophoresis and caused band shifting, thereby enabling a determination of activity (that is, the refolding rate).

### (b) The polymerase assay

Poly (dA) oligo (dT)₁₂₋₁₈ or DNase I-activated calf thymus DNA was used as the template DNA. The reaction solution had a composition (final concentration) of 50 mM TrisHCl pH 7.5 · 1 mM DTT · 15% glycerol · 5 mM MgCl₂ · 0.5 µM dTTP (cold) (thymidine triphosphate) · [³H]-dTTP (5 mCi/mL:100-500 cpm/pmol). The protein (enzyme) sample solution was first added to and suspended in 10 µL reaction solution that was twice as concentrated as that given above followed by incubation for 1 hour at 37°C, after which the reaction was stopped by holding on ice.

The reaction solution was then dripped onto DE81 paper that had been cut into a square. After drying, this was transferred to a beaker and was washed in order to dissolve and remove the unreacted dTTP. This wash consisted of first 3X with 5% aqueous disodium hydrogen phosphate solution, then 3X with distilled water, then 2X with ethanol, and was followed by drying. The dry DE81 paper obtained in this manner was placed in a scintillator-containing vial and the radioactivity (cpm) was measured with a scintillation counter. Stronger activity by the enzyme sample resulted in greater incorporation of radioisotope-labeled dTTP in the thereby synthesized DNA and thus in greater radioactivity, and the protein activity was determined on this basis.

### (c) Measurement of lysozyme activity

The bacteria M. lysodeikticus was selected as the substrate and was suspended in 50 mM phosphate buffer to prepare a substrate solution with a concentration of 0.16 mg/mL. 20 µL of the protein (lysozyme enzyme) solution was added to 480 µL of this substrate solution followed by incubation for 30 minutes at room temperature. This was followed by measurement of the absorbance at a wavelength of 450 nm.

Lysozyme has the capacity to degrade the cell wall of bacteria, and as a result the higher this capacity, that is, the activity, the greater the decline in absorbance. 1 unit of lysozyme activity was defined as a decline in absorbance at 450 nm of 0.001 per minute.

### (d) Measurement of the topoisomerase I activity

0.5 µg supercoiled pBR322 and the topoisomerase I protein were suspended in reaction buffer (10 mM TrisHCl pH 7.5, 150 mM NaCl, 5 mM beta-mercaptoethanol, 0.5 mM EDTA). After incubation for 30 minutes at 37°C, 0.1% SDS was added to stop the reaction. 0.5 µg/mL proteinase K was then added followed by incubation for 30 minutes at 37°C in order to degrade the topoisomerase I protein in the solution. The solution was subsequently subjected to electrophoresis on 1% (w/v) agarose and the DNA was stained with 0.5 µg/mL ethidium bromide. The topoisomerase I activity was measured based on observation of an upward shifted DNA band with a UV trans illuminator.

The activity (refolding rate) and protein recovery rate, which are the results for the subject examples obtained by the procedures and protocols described above, are shown in Tables 9, 10, and 13 together with the results for the comparative examples. As shown by the examples, activity native to the proteins, for example, the DNA binding activity, is produced by refolding. The refolding agent according to the present invention is useful as a highly versatile, highly generalizable refolding agent that is applicable to a variety of denatured and deactivated proteins and proteins that have an as yet unformed higher order structure; however, the application thereof is not limited to the proteins shown in the examples and the refolding agent according to the present invention can be applied to any protein.

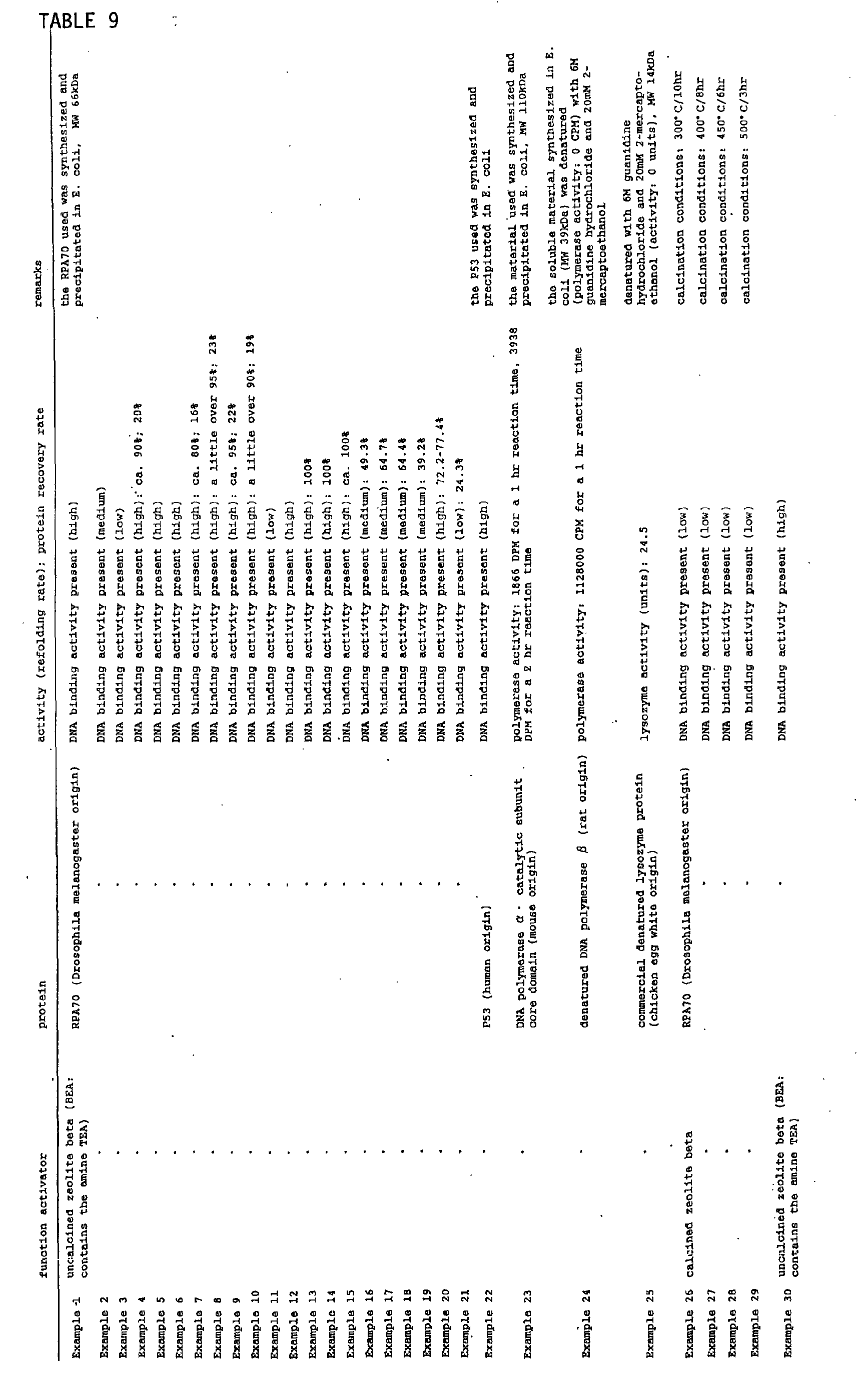

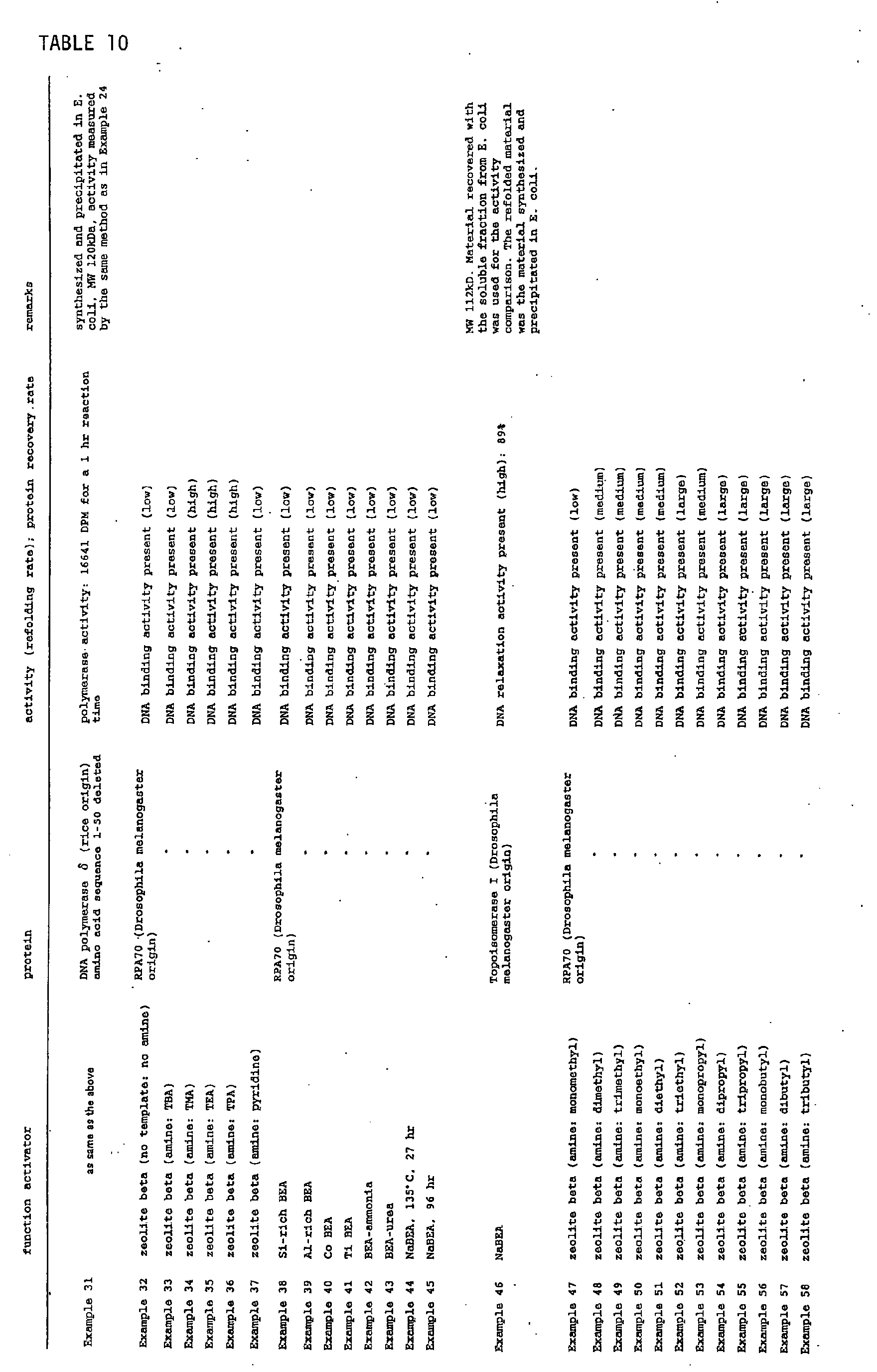

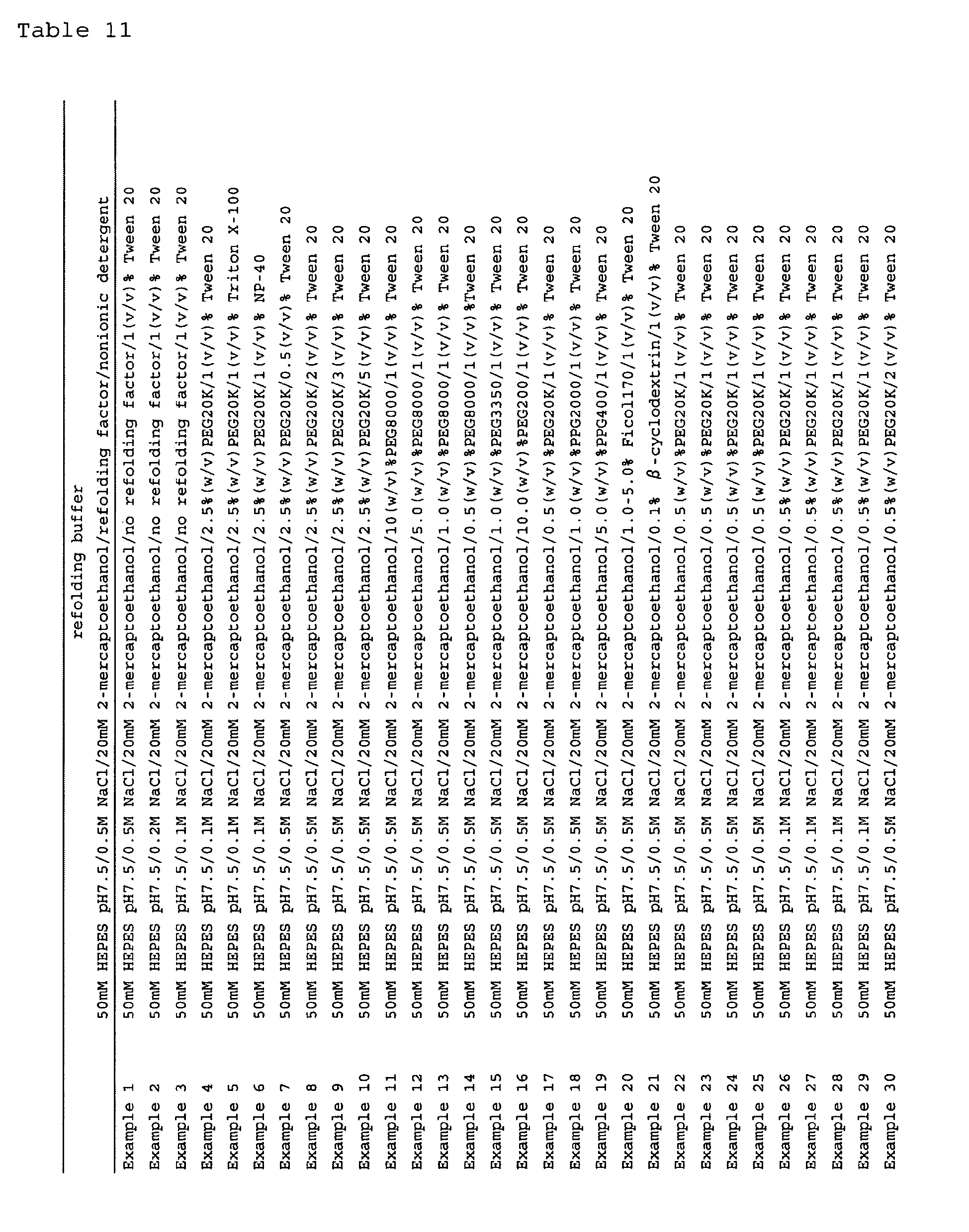

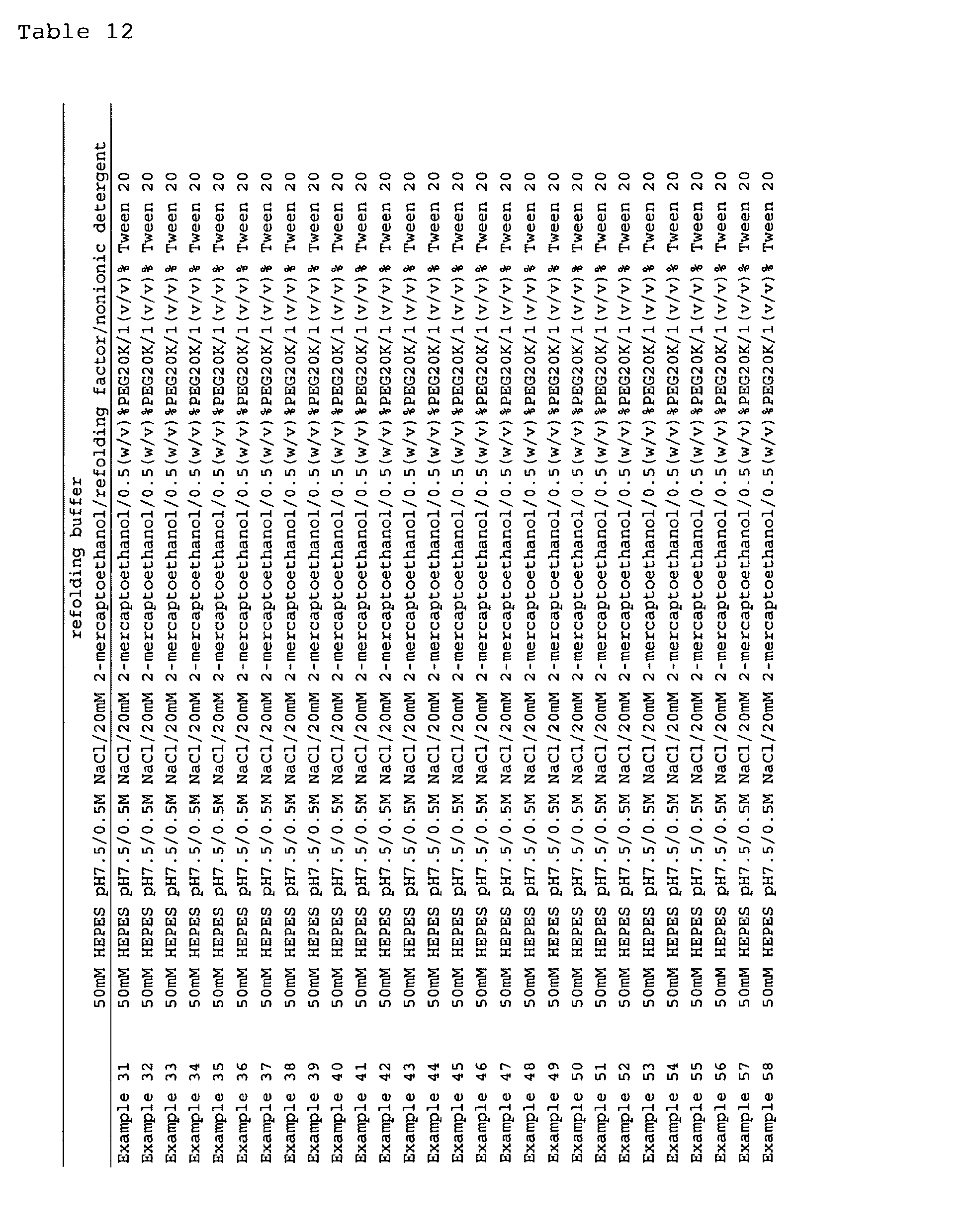

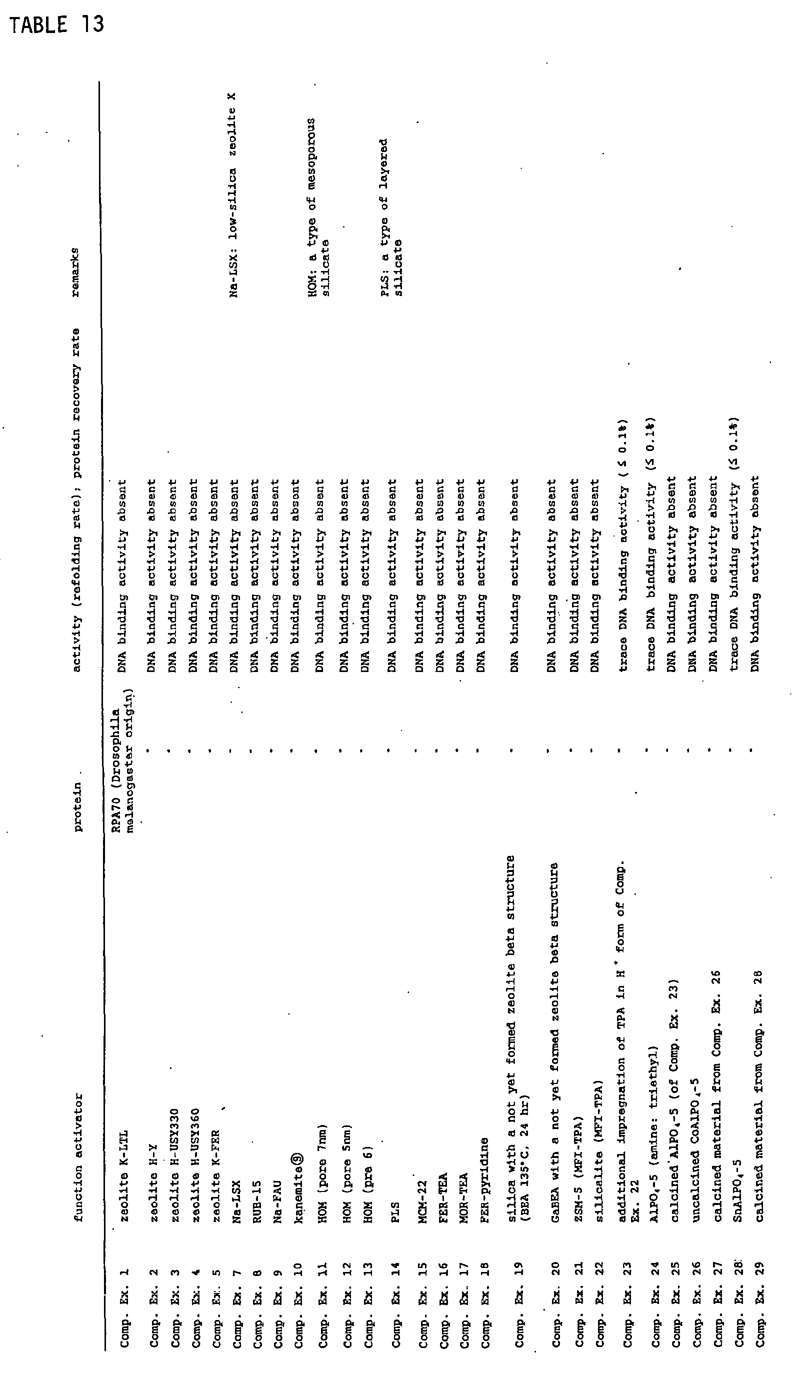

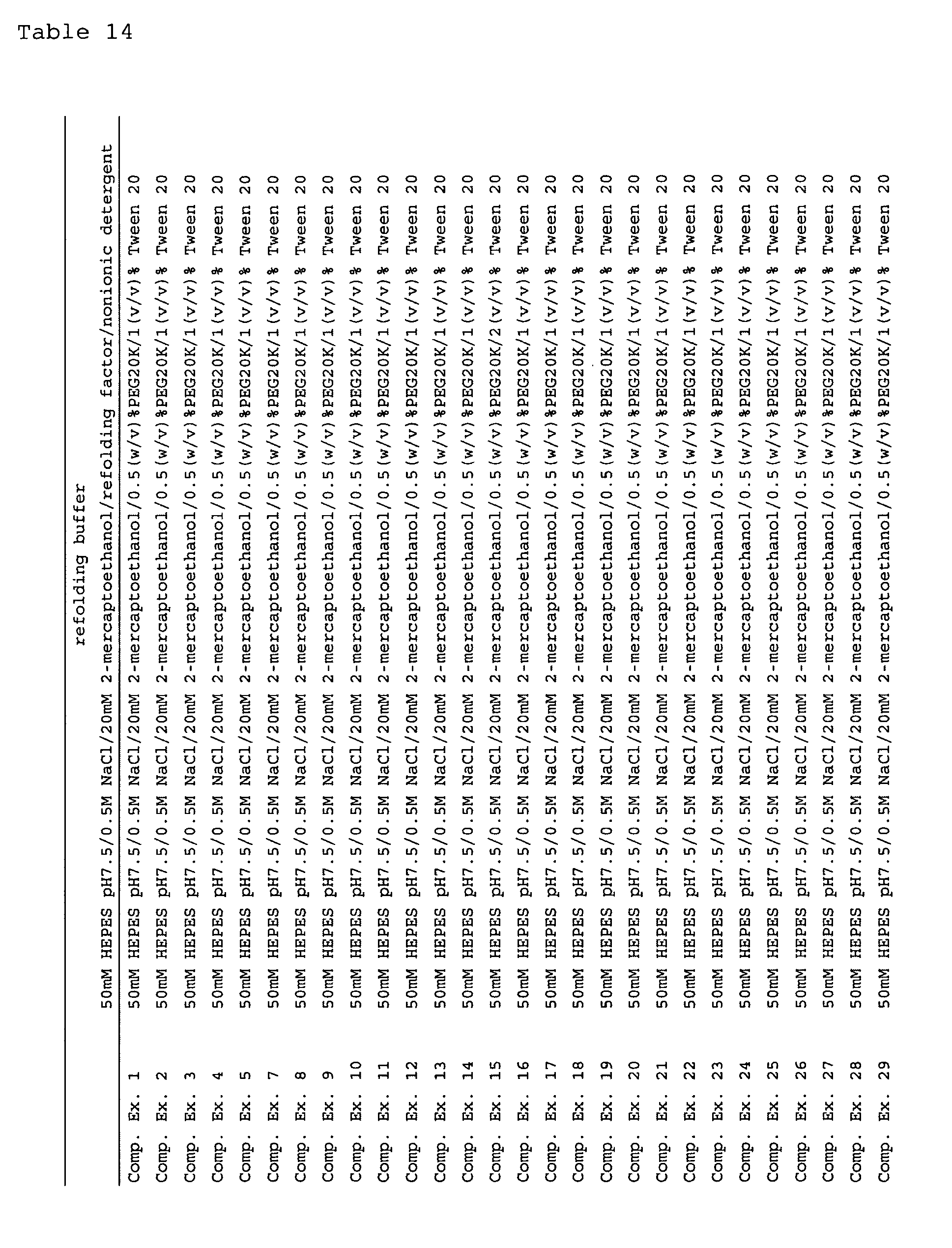

### Examples and Comparative Examples for the Fourth Aspect of the Present Invention

These examples provided below describe the activation of a function of protein produced by an Escherichia coli expression system and denatured protein; however, the present invention is neither limited to nor restricted by these examples.

### 1) Preparation of materials

### (a) The refolding moldings

Refolding moldings were fabricated by the following methods: conversion to zeolite beta by dry conversion and solid-phase conversion of an amorphous silica · alumina molding and an amorphous silica · alumina film coated on the surface of an inorganic ceramic support; deposition and immobilization of zeolite beta by in situ synthesis on filter paper and on the surface of an inorganic ceramic support; immobilization of zeolite beta on a support surface using an adhesive; and immobilization of zeolite beta utilizing an adhesive surface, such as adhesive tape. In the latter two methods, which employ an adhesive and an adhesive surface, the moldings were fabricated using zeolite beta that had been synthesized inhouse in advance, or using the commercially acquired product, and using various zeolite betas obtained by modifying the preceding by, for example, ion exchange.

### (b) The denatured protein solutions

RPA70 (Drosophila melanogaster origin), P53 (human origin), and so forth were used as the target proteins for activation.

### (c) The refolding buffer

A solution with the composition 50 mM HEPES pH 7.5/0.5 M NaCl/20 mM 2-mercaptoethanol/0.5% (w/v) polyethylene glycol 20000 (refolding factor) /1% (v/v) Tween 20 (detergent) was generally used as the refolding buffer.

### 2) Activity measurement protocols

Methods appropriate to the action of the protein used were employed for the activity measurements. Specifically, the four types of measurements described below, i.e., a gel shift assay, a polymerase assay, lysozyme activity measurement, and measurement of the topoisomerase I activity, were carried out.

### (a) The gel shift assay

1 pmol radioisotope-labeled DNA oligonucleotide and the refolded protein were incubated for 30 minutes on ice in a solution with a composition of 25 mM HEPES pH 7.4 · 50 mM KCl · 20% glycerol · 0.1% NP-40 · 1 mM DTT · 1 mg/mL bovine serum albumin. This was followed by electrophoresis at 4°C on 4.5% polyacrylamino gel using 0.5× TBE buffer.

When DNA binding to the protein was present (that is, when activity was present), the protein underwent binding to the DNA, which slowed down electrophoresis and caused band shifting, thereby enabling a determination of activity (that is, the refolding rate).

### (b) The polymerase assay

Poly (dA) oligo (dT)₁₂₋₁₈ or DNase I-activated calf thymus DNA was used as the template DNA. The reaction solution had a composition (final concentration) of 50 nmM TrisHCl pH 7.5 · 1 mM DTT · 15% glycerol · 5 mM MgCl₂ · 0.5 µM dTTP (cold) (thymidine triphosphate) · [³H]-dTTP (5 mCi/mL:100-500 cpm/pmol). The protein (enzyme) sample solution was first added to and suspended in 10 µL reaction solution that was twice as concentrated as that given above followed by incubation for 1 hour at 37°C, after which the reaction was stopped by holding on ice.

The reaction solution was then dripped onto DE81 paper that had been cut into a square. After drying, this was transferred to a beaker and was washed in order to dissolve and remove the unreacted dTTP. This wash consisted of first 3X with 5% aqueous disodium hydrogen phosphate solution, then 3X with distilled water, then 2X with ethanol, and was followed by drying. The dry DE81 paper obtained in this manner was placed in a scintillator-containing vial and the radioactivity (cpm) was measured with a scintillation counter. Stronger activity by the enzyme sample resulted in greater incorporation of radioisotope-labeled dTTP in the thereby synthesized DNA and thus in greater radioactivity, and the protein activity was determined on this basis.

### (c) Measurement of lysozyme activity

The bacteria M. lysodeikticus was selected as the substrate and was suspended in 50 mM phosphate buffer to prepare a substrate solution with a concentration of 0.16 mg/mL. 20 µL of the protein (lysozyme enzyme) solution was added to 480 µL of this substrate solution followed by incubation for 30 minutes at room temperature. This was followed by measurement of the absorbance at a wavelength of 450 nm. Lysozyme has the capacity to degrade the cell wall of bacteria, and as a result the higher this capacity, that is, the activity, the greater the decline in absorbance. 1 unit of lysozyme activity was defined as a decline in absorbance at 450 nm of 0.001 per minute.

### (d) Measurement of the topoisomerase I activity

0.5 µg supercoiled pBR322 and the topoisomerase I protein were suspended in reaction buffer (10 mM TrisHCl pH 7.5, 150 mM NaCl, 5 mM beta-mercaptoethanol, 0.5 mM EDTA). After incubation for 30 minutes at 37°C, 0.1% SDS was added to stop the reaction. 0.5 µg/mL proteinase K was then added followed by incubation for 30 minutes at 37°C in order to degrade the topoisomerase I protein in the solution. The solution was subsequently subjected to electrophoresis on 1% (w/v) agarose and the DNA was stained with 0.5 µg/mL ethidium bromide. The topoisomerase I activity was measured based on observation of an upward shifted DNA band with a UV trans illuminator.

### Refolding protocol example 1

0.5 mL of the above-described denatured protein (RPA70) solution (concentration 0.5 to 1.0 mg/mL) was dripped onto a film comprising zeolite beta powder spread over and immobilized on the adhesive surface of a commercially available tape (Cellotape®), thereby soaking the film surface with the solution. After standing for a while in order to ensure adsorption by the protein onto the refolding molding, the solution was shaken off and the film surface was washed four times with distilled water in order to completely remove the protein denaturant.

1 mL refolding buffer (prepared from 50 mM HEPES pH 7.5, 0.5 M NaCl, 20 mM 2-mercaptoethanol, refolding factor, and nonionic detergent) was then dripped onto the refolding molding and the protein adsorbed on the refolding molding was desorbed and eluted. The refolding molding was withdrawn and the solution that remained was transferred to a new Eppendorf tube and the activity was measured by the gel shift assay described above: activity was present, which confirmed that the RPA70 had undergone refolding.

### Refolding protocol example 2

Denatured RPA70 protein was refolded by entirely the same procedures and protocol as described above for protocol example 1, with the exception that this refolding protocol example 2 used a two-sided zeolite beta film molding fabricated using a commercially available two-sided adhesive tape. Activity was seen in the gel shift assay, which confirmed that refolding had occurred.

### Refolding protocol example 3

Denatured RPA70 protein was refolded by entirely the same procedures and protocol as described above for protocol example 1, with the exception that this refolding protocol example 3 used a molding fabricated by coating the surface of a commercially available porous alpha-alumina tube (cylindrical, length = 5 cm, opening diameter = 5 mm) with zeolite beta by in situ synthesis. Activity was seen in the gel shift assay, which confirmed that refolding had occurred.

### Refolding protocol comparative example 1

Refolding of denatured RPA70 protein used in refolding protocol examples 1 to 3 was carried out using finely divided zeolite beta powder. The protocol for this is described below. As compared to the use of a refolding molding according to the present invention, no fewer than 3 centrifugal separation steps were carried out, requiring the repetition of supernatant removal and washing each time, and as a consequence this protocol based on finely divided zeolite beta powder was very cumbersome.

100 mg finely divided zeolite beta powder was introduced into a 1.5 mL Eppendorf tube followed by the addition of 0.5 mL 6 M guanidine hydrochloride · 20 mM trisaminomethane trihydrochloride (TrisHCl) pH 7.5 · 0.5 M NaCl · 20 mM 2-mercaptoethanol and suspension. To this was then added 6 M guanidine hydrochloride · 20 mM 2-mercaptoethanol followed by holding for one hour on ice, after which 0.5 mL of the denatured RPA70 protein solution (concentration from 0.5 to 1.0 mg/mL) was added. In order to ensure adsorption of the protein on the finely divided zeolite beta powder, this mixture was stirred for 1 hour with a Rotary Culture RCC-100 (Iwaki Glass Co., Ltd.) placed in a cold room.

The finely divided zeolite beta powder was then sedimented by centrifugation for 5 seconds at 10000 × g and the supernatant was removed. In order to completely remove the protein denaturant from the sedimented finely divided zeolite beta powder, it was washed 4 times with 1 mL 20 mM TrisHCl pH 7.5 · 20 mM 2-mercaptoethanol (or water) followed by centrifugation for 5 seconds at 10000 × g and discard of the supernatant thereby produced. The remaining finely divided zeolite beta powder was suspended by the addition thereto of 1 mL refolding buffer (comprising 50 mM HEPES pH 7.5, 0.5 M NaCl, 20 mM 2-mercaptoethanol, refolding factor, and nonionic detergent) .

In order to desorb and elute the protein adsorbed on the finely divided zeolite beta powder, this suspension was again stirred in the cold with the Rotary Culture RCC-100 (Iwaki Glass Co., Ltd.). The finely divided zeolite beta powder was thereafter sedimented by centrifugation for 5 seconds at 10000 × g, and the protein-containing supernatant was transferred to a new Eppendorf tube and this was used for activity measurement by the gel shift assay. Activity was observed, which confirmed refolding of the RPA70.

As shown in the preceding examples, activity native to the protein, for example, DNA binding activity, is rapidly produced by a very simple protocol. The refolding molding according to the present invention is useful as a highly versatile, highly generalizable refolding molding that is applicable to a variety of denatured and deactivated proteins and proteins that have an as yet unformed higher order structure; however, the application thereof is not limited to the proteins shown in the examples and the refolding molding according to the present invention can be applied to any protein.

### INDUSTRIAL APPLICABILITY

As has been described hereinabove, the present invention relates, inter alia, to a method for activating a function of an inactive protein. The present invention enables the generation by refolding of a native function or activity of protein produced, for example, by an Escherichia coli expression system, that is inactive due to an as yet unformed higher order structure, or protein deactivated due to a change in conformation for some reason. This method is useful as a highly efficient method for refolding inclusion bodies. It can provide an efficient, versatile, and generalizable method that provides a high refolding rate and that can be applied to a variety of proteins. The zeolite beta of the function activator used by the present invention is inexpensive and can also be used repeatedly. This method can be applied to the refolding of a variety of conformation-disordered proteins, including large proteins with a molecular weight in excess of 100,000. For example, by combining the method according to the present invention with a protein synthesis process that employs an Escherichia coli expression system, it becomes possible to elaborate a novel process for manufacturing active protein that produces protein having a controlled higher order structure and expressing a native function inherent to the protein.

The present invention additionally provides an efficient, versatile, and generalizable refolding kit that has a high refolding rate and that is applicable to a variety of proteins. The present invention also provides a method for using this refolding kit. The zeolite beta constituting the refolding agent that is an essential component of the kit according to the present invention is inexpensive and can also be used repeatedly. This refolding kit has the ability to refold a variety of conformation-disordered proteins, including large proteins with a molecular weight in excess of 100,000. Accordingly, as a further development, through the combination, for example, of the kit according to the present invention and its method of use with a process of protein synthesis using an Escherichia coli expression system, a novel process and system for manufacturing active protein can be devised that produces protein having a controlled higher order structure and a native function inherent to the protein.

The present invention also relates to a function activator and a function-activating molding for inactive protein. The method using the function activator according to the present invention is useful as a highly efficient method for refolding inclusion bodies. An efficient refolding agent is provided that is versatile and generalizable and that has a high refolding rate and that is applicable to a variety of proteins. The zeolite beta constituting the refolding agent according to the present invention is inexpensive and can be used repeatedly. The refolding agent according to the present invention has the ability to refold a variety of conformation-disordered proteins, including large proteins with a molecular weight in excess of 100,000. Accordingly, as a further development, through the combination, for example, of the refolding agent according to the present invention and its method of use with a process of protein synthesis using an Escherichia coli expression system, a novel process and system for manufacturing active protein can be devised that produces protein having a controlled higher order structure and a native function inherent to the protein.

## Claims

1. A method of activating the function of a protein that is inactive due to a disordered higher order structure, comprising
bringing the protein into a state that can express a function inherent to the protein, by bringing the protein into contact with zeolite beta.

2. The method according to claim 1, wherein the protein is brought into contact with the zeolite beta in the presence of a protein denaturant, a surfactant, and/or a refolding buffer.

3. The method according to claim 1, wherein the protein that is inactive due to a disordered higher order structure is a protein that is produced by an Escherichia coli expression system.

4. The method according to claim 1, wherein the protein that is inactive due to a disordered higher order structure is a protein that is deactivated due to its thermal history.

5. The method according to claim 1, wherein the protein is adsorbed to the zeolite beta by mixing with a solution that contains the zeolite beta or by introduction onto a column packed with the zeolite beta and is then desorbed from the zeolite beta.

6. A method for reforming the core structure of a protein, comprising
refolding the conformation of a protein that is inactive due to a disordered higher order structure by bringing the protein into contact with zeolite beta.

7. A method for producing an active protein, comprising
refolding the conformation of a protein that is inactive due to a disordered higher order structure by bringing the protein into contact with zeolite beta, thereby producing a protein that has a controlled higher order structure and an activated native function inherent to the protein.

8. The method for producing a protein according to claim 7, comprising
refolding the conformation of an inactive protein produced by Escherichia coli that incorporates the genetic code responsible for the synthesis of a target protein, by bringing the inactive protein into contact with zeolite beta.

9. A protein refolding kit that is a reagent kit used in a protein function activation (refolding) protocol or step that modulates the higher order structure of a protein that is inactive due to a disordered higher order structure, thereby activating the protein, wherein the protein refolding kit contains a refolding agent comprising zeolite with the BEA structure (zeolite beta) as a constituent.

10. The refolding kit according to claim 9, wherein the kit has protein denaturant, pH regulator, and refolding agent comprising the zeolite beta as basic constituent components and additionally comprises a combination that contains at least one selection from agents that inhibit the formation of protein S-S bridges, surfactants, and refolding factors.

11. The refolding kit according to claim 9 or 10, wherein the framework structure of the zeolite beta contains silicon, oxygen, and at least one element other than silicon and oxygen.

12. The refolding kit according to any of claims 9 to 11, wherein the framework structure of the zeolite beta comprises only silicon and oxygen or only silicon and aluminum and oxygen.

13. The refolding kit according to any of claims 9 to 12, wherein the zeolite beta contains an ammonium species.

14. The refolding kit according to claim 13, wherein the ammonium species is ammonium ion, an organic amine, and/or an acid amide.

15. The refolding kit according to claim 14, wherein the organic amine is a tetraalkylammonium.

16. The refolding kit according to any of claims 10 to 15, wherein the protein denaturant in the kit is guanidine hydrochloride.

17. The refolding kit according to any of claims 10 to 16, wherein the pH regulator in the kit is trisaminomethane trihydrochloride (TrisHCl) and/or 4-(2-hydroxyethyl)-1-piperazineethanesulfonic acid (HEPES).

18. The refolding kit according to any of claims 10 to 17, wherein the agent in the kit that inhibits the formation of protein S-S bridges is 2-mercaptoethanol, dithiothreitol, cystine, or thiophenol.

19. The refolding kit according to any of claims 10 to 18, wherein the surfactant and refolding factor in the kit are at least one selection from polyethylene glycol, Ficol170, Ficol1400, polyphosphoric acid, sodium dodecyl sulfate (SDS), sucrose, glucose, glycerol, inositol, cyclodextrin, amylose, Dextran T-500, Tween 20, Tween 40, Tween 60, NP-40, SB3-14, SB12, CTAB, and Triton X-100.

20. The refolding kit according to any of claims 9 to 15, wherein the kit comprises said refolding agent, guanidine hydrochloride, TrisHCl, 2-mercaptoethanol, and a solution (the refolding buffer) comprising HEPES, alkali halide, 2-mercaptoethanol, refolding factor, and surfactant, or the kit comprises the refolding buffer, the refolding agent, guanidine hydrochloride, TrisHCl, 2-mercapethanol, and alkali halide.

21. A protein refolding agent that has a protein refolding action that modulates the higher order structure of and activates a protein that is inactive due to a disordered higher order structure, the protein refolding agent comprising zeolite with the BEA structure (zeolite beta).

22. The refolding agent according to claim 21, that carries out protein refolding in the presence of a protein denaturant, a surfactant, and/or a refolding buffer.

23. The refolding agent according to claim 21, wherein the protein that is inactive due to a disordered higher order structure is a protein that is produced by an Escherichia coli expression system.

24. The refolding agent according to claim 21, wherein the protein that is inactive due to a disordered higher order structure is a protein that is deactivated due to its thermal history.

25. The refolding agent according to claim 21, wherein the zeolite beta contains ammonium ion, an organic ammonium ion, and/or urea.

26. The refolding agent according to claim 25, wherein the organic ammonium ion is a mono-, di-, tri-, and/or tetraalkylammonium ion (where the alkyl group is methyl, ethyl, propyl, or butyl).

27. The refolding agent according to claim 21, wherein the framework structure of the zeolite beta comprises oxygen and at least one element other than oxygen.

28. The refolding agent according to claim 27, wherein the framework structure of the zeolite beta comprises silicon and oxygen or silicon, aluminum, and oxygen.

29. The refolding agent according to any of claims 21 to 28, that manifests a protein refolding action through contact with a protein dispersed in a solution.

30. The refolding agent according to any of claims 21 to 29, that causes refolding of the protein by a procedure in which said protein in a solution is adsorbed by mixing with the refolding agent or by introduction onto a column packed with the refolding agent and thereafter is desorbed.

31. A refolding molding comprising a molding that contains zeolite with the BEA structure (known as zeolite beta) that has the capacity, denoted as a refolding activity, to modulate and activate the higher order structure of a protein that is inactive due to a disordered higher order structure.

32. The refolding molding according to claim 31, wherein the molding comprises zeolite beta or zeolite beta and a substrate that supports the zeolite beta.

33. The refolding molding according to claim 31, that manifests a refolding activity upon contact with a protein.

34. The refolding molding according to claim 31, that carries out the refolding of a protein in the presence of a protein denaturant, a surfactant, and/or a refolding buffer.

35. The refolding molding according to claim 31, wherein the protein that is inactive due to a disordered higher order structure is a protein that is produced by an Escherichia coli expression system.

36. The refolding molding according to claim 31, wherein the protein that is inactive due to a disordered higher order structure is a protein deactivated due to its thermal history.

37. The refolding molding according to claim 31, wherein the zeolite beta contains ammonium ion and/or organic ammonium ion.

38. The refolding molding according to claim 37, wherein the organic ammonium is a mono-, di-, tri-, and/or tetraalkylammonium ion (where the alkyl group is methyl, ethyl, propyl, or butyl).

39. The refolding molding according to claim 31, wherein the framework structure of the zeolite beta comprises oxygen and at least one element other than oxygen.

40. The refolding molding according to claim 39, wherein the framework structure of the zeolite beta comprises silicon and oxygen or silicon, aluminum, and oxygen.

41. The refolding molding according to any of claims 31 to 40, that manifests a protein refolding activity through contact with a protein dispersed in a solution.

42. The refolding molding according to any of claims 31 to 41, that has a function that causes refolding of the protein by a procedure in which the protein is adsorbed to the molding by mixing the protein in a solution with the refolding molding or by flowing or dripping the protein in a solution onto the molding and thereafter is desorbed.
